# EUROPEAN PATENT APPLICATION

(11) **EP 3 841 901 A1**
(43) Date of publication of application: **30.06.2021**
(21) Application number: 19864012.0
(22) Date of filing: 17.09.2019
(51) Int. Cl.: A41D 13/05, A41D 1/06

(54) **BOTTOM GARMENT**

(30) Priority: 25.09.2018 JP 2018178710
(71) Applicant: Toray Industries, Inc., Tokyo, 103-8666 (JP)
(72) Inventor: TAKADA, Nana, Otsu-shi, Shiga 520-2141 (JP); KAJIYAMA, Hiroshi, Otsu-shi, Shiga 520-2141 (JP); MIYAMURA, Takako, Tokyo 101-0032 (JP); MIZUNO, Tadayuki, Tokyo 101-0032 (JP)
(74) Representative: Prüfer & Partner mbB Patentanwälte · Rechtsanwälte
(86) International application number: PCT/JP2019/036329
(87) International publication number: WO 2020/066740

(57) **Abstract**

The purpose of the present invention is to provide a bottom wear which has a cylindrical part on the back part of a waist-encircling section and inside the bottom wear and in which a protection band for lumbago can be attached to a bottom wear such as work trousers suitable for use with a protection band for lumbago for tightening a waist of a wearer. The bottom wear allows the wearer to quickly complete work for attaching the protection band for lumbago to the cylindrical part. The means for achieving the purpose is a bottom wear including a waist-encircling section. In the bottom wear, cylindrical part including a cloth of bottom wear and a planar material different from the cloth, or a cylindrical part including a planar material different from the cloth of bottom wear is present at a back portion of the waist-encircling section and inside the bottom wear. The cylindrical part is disposed in such a manner that the axis direction of the cylindrical part is substantially parallel to the waist-encircling direction of the waist-encircling section. One slit is present at the substantially central part of the front portion of the cylindrical part. The slit is disposed in such a manner that the longitudinal direction is substantially parallel to the wearer's stature direction. The ratio (W2/W1) of the width of the slit in the longitudinal direction (W2) to the width of the cylindrical part (W1) is 0.70 to 1.00.

## Description

### TECHNICAL FIELD

The present invention relates to a bottom wear.

### BACKGROUND ART

Among occupational diseases, low back pain is one of diseases incident to workers during working, and is spreading in various types of occupations. Examples of occupational workers who have a high risk of being attacked by low back pain include transportation operators who are engaged in handling heavy weight articles and in long-distance driving and medical workers working for assistance of inpatients and treating emergency patients, and recently, the number of workers relating to services in social welfare facilities for care of the elderly and the like and suffering from low back pain has considerably increased.

The number of occupational workers suffering from low back pain tends to increase as an age becomes older, but only a few people take a measure at an initial stage of low back pain, and quite a few take no step until the low back pain gets worse. In addition, mainly developed countries are facing a problem with a decreased working-age population due to low birthrate and longevity. In addition, there is an increasing number of senior workers of 65 years old or older and woman workers in the light of support of a society. Such being the case, importance of maintenance and promotion of health of valuable workers is increasing more than before. Also in case of working uniforms, a viewpoint of keeping health of workers positively becomes important in addition to conventional functions such as safety, mobility and discrimination.

In order to prevent low back pain, it has been considered effective to tighten a waist portion of a wearer, in particular a portion including an upper part of a pelvis of the wearer with a protection band for lumbago, and increase an abdominal pressure. The purpose of this is to decrease a burden of a waist by forming an abdominal cavity into a shape like a rugby ball to work as a support. However, there is a problem that since such a protection band for lumbago is mostly worn under a wear, and must be worn on a body after taking off the wear, fitting thereof is troublesome and that also when adjusting the fastening of the waist protection band after wearing it, since a degree of the fastening must be adjusted after the wear is taken off or loosened, this work is troublesome.

Therefore, there is known a bottom wear which has a cylindrical part on a back surface part of a waist-encircling section and inside the bottom wear and in which a protection band for lumbago can be integrally attached to a bottom wear such as work trousers (see Document 1). The bottom wear has a configuration in which even if a wearer whose waist portion is tightened by a protection band for lumbago repeatedly performs an expansion and contraction motion in which the posture is changed from an upright posture to a crouching posture or from a crouching posture to an upright posture, the underwear worn by the wearer can be inhibited from sliding in a wearer's stature height direction with respect to the waist portion of the wearer as the wearer slides in the wearer's stature height direction with respect to the waist. This solves the problem that the wearer feels uncomfortable as the posture of the wearer changes as described above.

### PRIOR ART DOCUMENT

### PATENT DOCUMENT

Patent Document 1: WO 2018/066504

### SUMMARY OF THE INVENTION

### PROBLEMS TO BE SOLVED BY THE INVENTION

However, according to the findings by the inventors, a bottom wear in which protections for lumbago can be integrally attached as disclosed in Patent Document 1 has a structure in which when a protection band for lumbago (hereinafter, sometimes referred to simply as a "band") is attached to a cylindrical part, the protection band for lumbago is inserted through an opening disposed at one of both ends of the cylindrical part, and the work for insertion of the protection band for lumbago tends to be work taking much time and a lot of energy due to occurrence of troubles such that an end of protection band for lumbago or the like is caught on a lateral surface inside the cylindrical part in the middle, so that the protection band for lumbago is stuck inside the cylindrical part. Further, occupational workers who have a high risk of being attacked by the low back pain, such as workers relating to transportation business, medical workers, and workers relating to services in social welfare facilities for care of the elderly and the like, often perform the work for insertion of the protection band for lumbago in limited dressing space. In addition, since mostly a plurality of persons change clothes at the same time, it is difficult to secure enough space for one person, and the persons try to change clothes quickly in order to provide dressing space for other persons. Thus, in a method in which a band is inserted through an opening disposed at one of both ends of a cylindrical part (i.e. a method in which one end of the band is inserted through one opening of the cylindrical part, and caused to pass toward the other opening of the cylindrical part to attach the band), the band may be stuck in the middle of the cylindrical part, or bent within the cylindrical part. In this case, the worker should insert his or her hand to the inside of the cylindrical part through the other opening of the cylindrical part to pull out the band. However, even when a hand is inserted through the other opening of the cylindrical part to pull out the band as mentioned above, it is difficult to insert the hand to a position at which the band is stuck if the distance over which the band passes (hereinafter, sometimes referred to as a "band passage distance"), such as a distance from one opening to the other opening of the cylindrical part. Due to the above-mentioned problems, the bottom wear disclosed in Patent Document 1, which cannot choose but employ a method in which a band is inserted through an opening disposed at one of both ends of the cylindrical part, has the problem that it takes much time for the wearer to complete attachment of the protection band for lumbago to the cylindrical part.

### SOLUTIONS TO THE PROBLEMS

For solving the above-described problems, the present invention discloses the following constitution and more preferable aspects.
1. A bottom wear including a waist-encircling section, wherein
   a cylindrical part including a cloth of bottom wear and a planar material different from the cloth, or
   a cylindrical part including a planar material different from the cloth of bottom wear is present at a back portion of the waist-encircling section and inside the bottom wear,
   the cylindrical part is disposed in such a manner that the axis direction of the cylindrical part is substantially parallel to the waist-encircling direction of the waist-encircling section,
   the cylindrical part includes only one slit satisfying the following conditions (1) and (2), and
   the slit is provided at the front portion of the cylindrical part and the substantially central part of the cylindrical part in a longitudinal direction:
   (1) the slit is provided in such a manner that the longitudinal direction of the slit is substantially orthogonal to the waist-encircling direction; and
   (2) the ratio of the width (W2) of the slit in the longitudinal direction to the width (W1) of the cylindrical part in a direction orthogonal to the waist-encircling direction (W2/W1) is 0.70 to 1.00.
2. The bottom wear according to the above, in which the static friction coefficient of the inner surface of the rear portion of the cylindrical part of the bottom wear is 0.2 to 1.0, and the stress at the front portion of the cylindrical part is 10 N/25 mm or less at 10% elongation of the cylindrical part in the width direction.
3. The bottom wear according to the above, in which the static friction coefficient of the surface of a part of the front portion of the cylindrical part, which is far from a human body, is 0.2 to 1.0.
4. The bottom wear according to the above, in which the static friction coefficient of the surface of a part of the front portion of the cylindrical part, which is close to a human body, is 0.2 to 1.0.
5. The bottom wear according to any one of items 1 to 4, in which the cylindrical part includes a cloth of bottom wear and a planar material different from the cloth, and
   the upper part and the lower part of the planar material are each continuously or intermittently connected to the cloth of bottom wear.
6. The bottom wear according to any one of items 1 to 4, in which the cylindrical part includes a planar material different from the cloth of bottom wear, and
   the upper part and the lower part of the cylindrical part are each continuously or intermittently connected to the cloth of bottom wear.
7. The bottom wear according to any one of the above, in which the planar material is a fabric.
8. A bottom wear with a band which includes the bottom wear set forth in any one of the above, and a protection band for lumbago.

### EFFECT OF THE INVENTION

It is possible to provide a bottom wear which has a cylindrical part on the back part of a waist-encircling section and inside the bottom wear and in which a protection band for lumbago can be attached to a bottom wear such as work trousers suitable for use with a protection band for lumbago for tightening a waist of a wearer. The bottom wear enables the wearer to quickly complete work for attaching the protection band for lumbago to the cylindrical part.

### BRIEF DESCRIPTION OF THE DRAWINGS

Fig. 1 is a view of a bottom wear according to a first embodiment of the present invention where the bottom wear is seen from the front and above.
Fig. 2 is a right side view of the bottom wear shown in Fig. 1.
Fig. 3 is a sectional view taken along line A-A' of the bottom wear shown in Fig. 1.
Fig. 4 is a front view of one example of a protection band for lumbago which can be attached to the bottom wear of the present invention.
Fig. 5 is a rear view of one example of a protection band for lumbago which can be attached to the bottom wear of the present invention.
Fig. 6 is a view of the bottom wear shown in Fig. 1, to which a protection band for lumbago is attached, where the bottom wear is seen from the front and above.
Fig. 7 is a sectional view taken along line B-B' of the bottom wear shown in Fig. 6, to which a protection band for lumbago is attached.
Fig. 8 is a view of a bottom wear according to a second embodiment of the present invention where the bottom wear is seen from the front and above.
Fig. 9 is a sectional view taken along line C-C' of the bottom wear shown in Fig. 8.
Fig. 10 is a view of the bottom wear shown in Fig. 8, to which a protection band for lumbago is attached, where the bottom wear is seen from the front and above.
Fig. 11 is a sectional view taken along line D-D' of the bottom wear shown in Fig. 10, to which a protection band for lumbago is attached.
Fig. 12 is a sectional view taken along line E-E' of the bottom wear shown in Fig. 2.
Fig. 13 is a right side view of the bottom wear shown in Fig. 1.
Fig. 14 is a right side view of the bottom wear shown in Fig. 8.
Fig. 15 is a view of a bottom wear having a structure in which a protection band for lumbago is completely hidden inside the bottom wear, where the bottom wear is seen from the front and above.
Fig. 16 is a view of the bottom wear shown in Fig. 15, to which a protection band for lumbago is attached, where the bottom wear is seen from the front and above.
Fig. 17 is a view of a bottom wear having a structure in which a protection band for lumbago can be partially hidden, where the bottom wear is seen from the front and above.
Fig. 18 is a view of the bottom wear shown in Fig. 17, to which a protection band for lumbago is attached, when the protection band for lumbago is partially hidden, where the bottom wear is seen from the front and above.
Fig. 19 is a view of the bottom wear shown in Fig. 17, to which a protection band for lumbago is attached, when the protection band for lumbago is not partially hidden but exposed on the front surface, where the bottom wear is seen from the front and above.
Fig. 20 is a view of a bottom wear used in Example 15, where the bottom wear is seen from the front and above.
Fig. 21 is a view of a bottom wear used in Comparative Example 2, where the bottom wear is seen from the front and above.
Fig. 22 is a view of a bottom wear used in Comparative Example 1, where the bottom wear is seen from the front and above.
Fig. 23 is a schematic view of the bottom wear shown in Figs. 1 and 3, where the bottom wear is shown in a first step of a procedure for attaching the protection band for lumbago to the cylindrical part.
Fig. 24 is a schematic view of the bottom wear shown in Figs. 1 and 3, where the bottom wear is shown in a second step of a procedure for attaching the protection band for lumbago to the cylindrical part.
Fig. 25 is a schematic view of the bottom wear shown in Figs. 1 and 3, where the bottom wear is shown in a third step of a procedure for attaching the protection band for lumbago to the cylindrical part.

### EMBODIMENTS OF THE INVENTION

The meanings of the terms used in the present invention are as follows.

Bottom wear: a wear worn on a lower body, e.g. pants such as slacks, jeans, chino pants, cargo pants and short pants, skirts and boiler suits.

Waist-encircling section: a part or the whole of a bottom wear which comes into direct or indirect contact with a portion around a body surface at a portion ranging from a lower part of a spinal column to a pelvis of a wearer at the time when the bottom wear is worn. The waist-encircling section may have at least one selected from the group consisting of a waist portion including a belt loop, side pockets, back pockets and front and back fasteners, and a rise portion of trousers.

Back portion: a part of a bottom wear which comes into direct or indirect contact with the back of a wearer.

Cylindrical part: a cylindrical part present on a waist-encircling section and a back portion of a bottom wear. The cylindrical part is present on the front part of the back portion of the bottom wear main body. The cylindrical part is not necessarily required to have a circular cross-section, and may have a hollow cross-section, or a cross-section which can be made hollow. The cylindrical part may be collapsed, or folded into a substantially planar shape. The axial direction of the cylindrical part is also a length direction of the cylindrical part.

Cylindrical part in the first embodiment of the present invention: a cylindrical part including a cloth of bottom wear and a planar material different from the cloth.

Cylindrical part in the second embodiment of the present invention: a cylindrical part including a planar material different from a cloth of bottom wear.

Invention of the first embodiment: an invention having a cylindrical part according to the first embodiment.

Invention of the second embodiment: an invention having a cylindrical part according to the second embodiment.

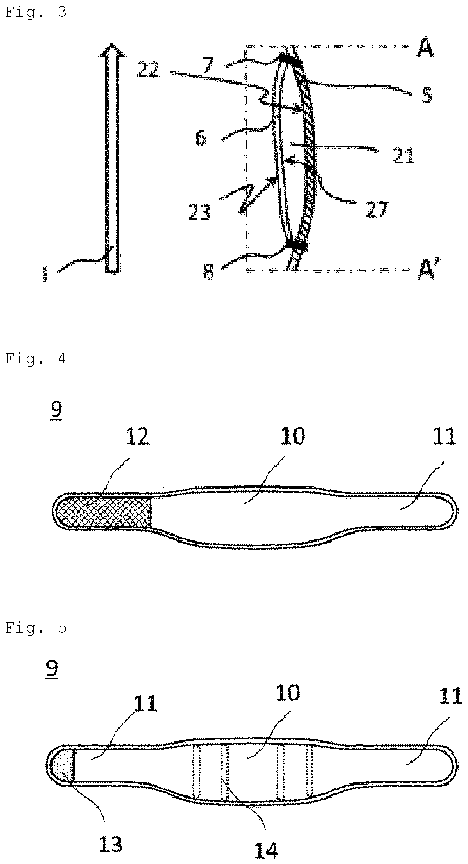

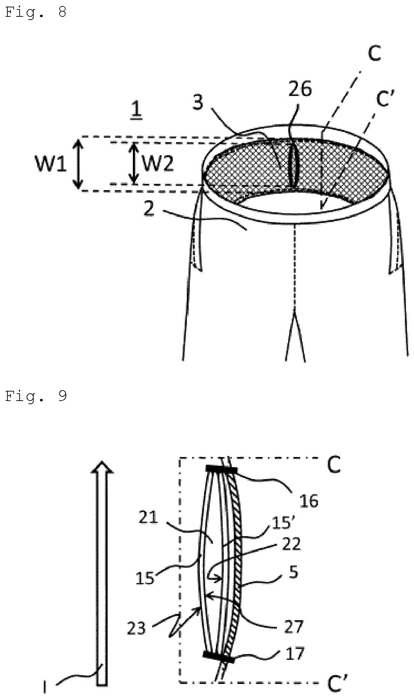

Rear portion of cylindrical part: The cylindrical part can be divided into two parts by the line between the upper end and the lower end of the cylindrical part in the direction of the wearer's height. Among the above-mentioned portions, the rear portion is a portion far from a wearer. In the first embodiment, the rear portion is a "cloth of bottom wear 5 on a back portion in a waist-encircling section" in Fig. 3. In the second embodiment, the rear portion is a "rear portion 15' of a cylindrical part" in Fig. 9. Hereinafter, it is sometimes referred to as a "rear portion of a cylindrical part".

Front portion of cylindrical part: The cylindrical part can be divided into two parts by the line between the upper end and the lower end of the cylindrical part in the direction of the wearer's height. Among the above-mentioned portions, the front portion is a portion close to a wearer. In the first embodiment, the front portion is a "front portion 6 of a cylindrical part" in Fig. 3. In the second embodiment, the front portion is a "front portion 15 of a cylindrical part" in Fig. 9. Hereinafter, it is sometimes referred to as a "front portion of a cylindrical part".

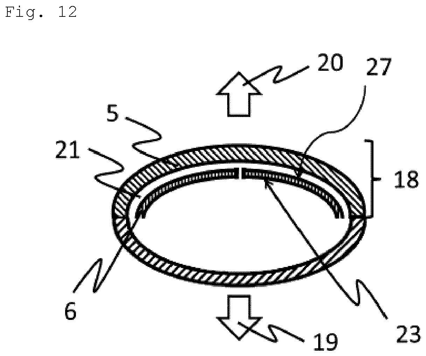

Front: a frontward direction of a wearer in a bottom wear (symbol 19 in Fig. 12).

Rear: a rearward direction of a wearer in a bottom wear (symbol 20 in Fig. 12).

Hereinafter, the present invention will be described with reference to the drawings, but the present invention is not limited to the following disclosure.

First, the bottom wear according to the first embodiment will be described.

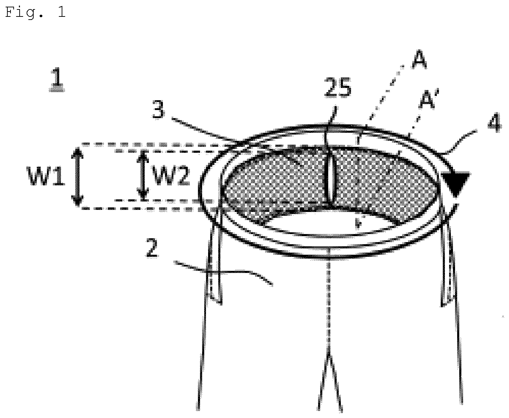

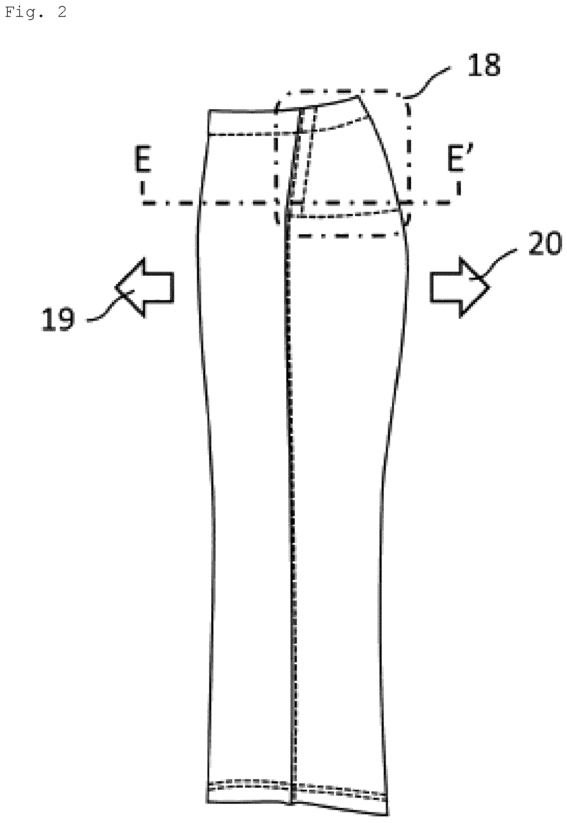

Fig. 1 is a view showing a part of the bottom wear as an example of the first embodiment, where the part of the bottom wear is seen from the front and above, and Fig. 2 is a right side view of the bottom wear shown in Fig. 1. The bottom wear 1 of the present invention has a waist-encircling section 2 around the bottom wear.

In addition, the waist-encircling section 2 of the bottom wear 1 according to the first embodiment has a cylindrical part 3 on a back portion 18 thereof. The cylindrical part 3 is disposed in such a manner that the axis direction of the cylindrical part is substantially parallel to a waist-encircling direction 4 of the waist-encircling section.

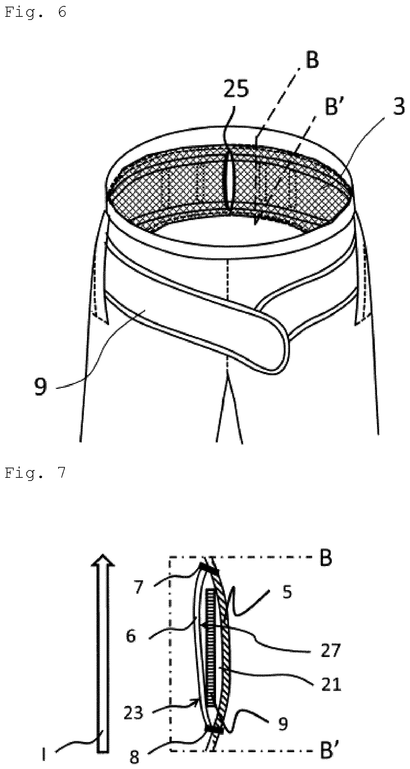

See Fig. 6. Inside the cylindrical part 3, a protection band for lumbago 9 can be inserted into the cylindrical part 3.

Next, Fig. 3 is a sectional view taken along line A-A' of the bottom wear shown in Fig. 1. As shown in Fig. 3, the cylindrical part 3 in Fig. 1 includes a bottom wear cloth 5 on a back portion in the waist-encircling section, and a planar material forming a cylindrical part front portion 6, and the planar material is disposed inside the bottom wear. The bottom wear cloth 5 is a cylindrical part rear portion in the present invention. In addition, a planar material is used for the cylindrical part front portion 6. As a result, a cylindrical part 3 including a cloth of bottom wear and a planar material different from the cloth is formed.

The upper end of the cylindrical part front portion 6 including the planar material is continuously or intermittently connected to the bottom wear cloth 5 by a locking portion 7 in the waist-encircling direction. Preferably, the upper end of the planar material forming the cylindrical part front portion 6 is connected to the waist-encircling section as in the configuration in Fig. 3 because it is possible to suppress a situation in which the toe or the like of a wearer is caught between the planar material and the bottom wear cloth 5 at the time of wearing the bottom wear of the present embodiment.

It is to be noted that the present invention is not limited to the configuration shown in Fig. 3. A portion slightly below the upper end of the planar material (a part situated below the upper end of the planar material) may be connected, i.e., the upper end of the planar material may be protruded above the locking portion.

In addition, in Fig. 3, the lower end of the planar material forming the cylindrical part front portion 6 is intermittently connected in a waist-encircling direction to the bottom wear cloth 5 on the back portion in the waist-encircling section by a locking portion 8. The reason why it is preferable that the planar material be connected at the lower end is as follows.

The lower end portion of the planar material can be fixed so as to extend along the inner surface of the cloth of bottom wear. As a result, a protection band for lumbago is neatly inserted into the cylindrical part. In addition, it is possible to suppress uncontrollable movement of the lower end portion of the planar material inside the bottom wear. It is possible to suppress bending of the planar material inside the bottom wear, and therefore a wearer can insert a protection band for lumbago into the cylindrical part while wearing the bottom wear.

The present invention is not limited to the configuration shown in Fig. 3. A portion slightly above the lower end of the planar material (a part situated above the lower end of the planar material) may be connected, i.e., the lower end of a fabric may be protruded below the locking portion.

In the bottom wear configured to have a cross-section as in Fig. 3 in the present invention, the locking portion 7 and the locking portion 8 are formed by stitch, and the upper part and the lower part of the fabric (planar material) are each intermittently connected in the waist-encircling direction to the bottom wear cloth on the back portion in the waist-encircling section. The connection is not required to be intermittent, and may be continuous. Preferably, the connection is performed at the upper end and the lower end of the planar material where a cylindrical part is formed. Here, examples of the intermittent locking portion include those obtained by stitch, an adhesive, welding, or locking with a hook-and-loop fastener or button, or the like. On the other hand, examples of the continuous locking portion include those obtained by stitch or welding. The stitch may be normal lock stitch, but is more preferably chain stitch such as flat seam stitch, two needles stitch or three needles stitch from the viewpoint of smoothing the texture to a person. In addition, an example of the configuration of the intermittent locking portion is not limited to the configuration of the locking portion of the bottom wear of the present embodiment shown in Fig. 1, and may be, for example, a configuration in which only one locking portion is provided at the lower end of both end portions of the cylindrical part in the axis direction of the cylindrical part.

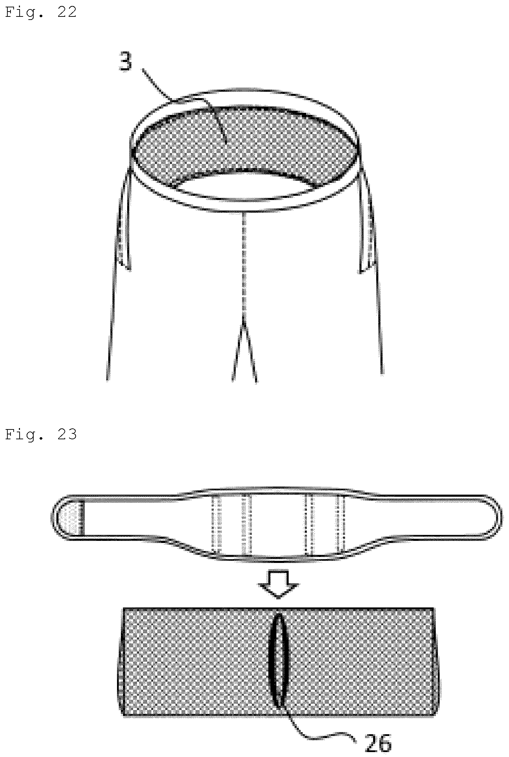

Further, only one slit 25 is disposed at the central part of the cylindrical part front portion 6 shown in Figs. 1 and 3, and the slit 25 is provided in such a manner that the longitudinal direction is substantially orthogonal to the waist-encircling direction. If there is no slit 25, the cylindrical part will be as shown in Fig. 22. In the structure of a cylindrical part as shown in Fig. 22, when a protection band for lumbago is attached, a worker inserts one end of the band through an opening disposed at one end of the cylindrical part, and causes the band to pass toward the other opening of the cylindrical part, so that the band is attached. In such an attachment method, the band may be stuck in the middle of the cylindrical part, or bent within the cylindrical part. In this case, the worker should insert his or her hand to the inside of the cylindrical part through the other opening of the cylindrical part to pull out the band. However, even when a hand is inserted through the other opening of the cylindrical part to pull out the band as mentioned above, it tends to be difficult to insert the hand to a position at which the band is stuck if the band passage distance such as a distance from one opening to the other opening of the cylindrical part is long. Due to the above-mentioned problems, a bottom wear having a configuration, which cannot choose but employ a method in which a band is inserted through an opening disposed at one of both ends of the cylindrical part, has the problem that it takes much time for the wearer to complete attachment of the protection band for lumbago to the cylindrical part. However, by providing the slit 25, the protection band for lumbago can be easily attached to the bottom wear in accordance with a procedure as shown in Figs. 23, 24, and 25.

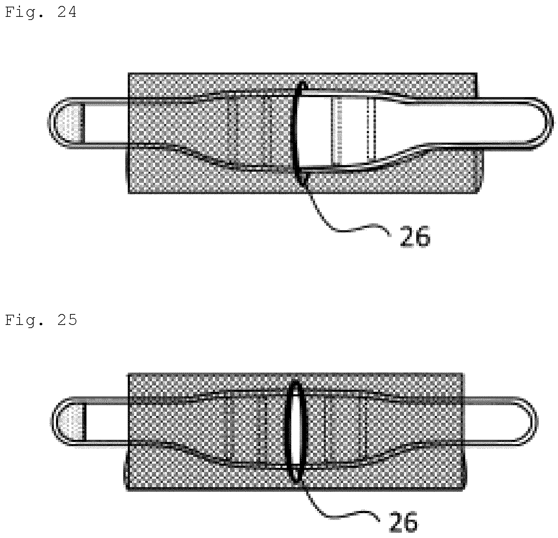

Specifically, first, one of the right and left ends of the protection band for lumbago with respect to the center of the back portion is caused to pass into the cylindrical part (Fig. 24), and the other end is caused to pass into the cylindrical part on the opposite side (Fig. 25), whereby the protection band for lumbago can be attached, so that it is possible to considerably reduce the time for inserting protection band for lumbago. As shown in Fig. 1, the cylindrical part is provided with only one slit 25. The reason for limiting the number of slits 25 to one is as follows. The slit in the bottom wear of the first embodiment is disposed for the purpose of facilitating work for inserting the protection band for lumbago through the slit and inserting the protection band for lumbago into the cylindrical part. Therefore, if the number of slits is two or more (for example slits A and B), the tip of the protection band for lumbago is caught in the slit B of the protection band for lumbago at the time of inserting the protection band for lumbago into the cylindrical part through the slit A, so that it is difficult for the band to smoothly enter through the slit A, resulting in rather expansion of time-consuming work. Thus, it tends to be impossible to obtain an effect of providing slits in the cylindrical part front portion. Further, as the number of slits increases, the time-consuming work expands as described above, so that the effect of providing slits in the cylindrical part front portion decreases. For the reason described above, the number of slits provided in the cylindrical part front portion is limited to one.

Further, in the present embodiment, the slit is provided in the front portion of the cylindrical part and in the substantially central part of the cylindrical part in the longitudinal direction. Here, it is preferable that the slit be disposed only in the front portion of the cylindrical part. Since the slit is disposed only in the front portion of the cylindrical part, i.e., there is no slit in the rear portion of the cylindrical part, it is possible to avoid exposure of the protection band for lumbago from the appearance of the waist-encircling section at the time when the protection band for lumbago is attached to the cylindrical part. In such a bottom wear, the protection band for lumbago is inhibited from being caught by projections or the like outside the bottom wear, and therefore a wearer can safely perform work. The reason why the position at which the slit is provided is set to the cylindrical part front portion and the substantially central part of the cylindrical part in the longitudinal direction is as follows. As described above, the slit in the bottom wear of the first embodiment is disposed for the purpose of facilitating work for inserting the protection band for lumbago through the slit and inserting the protection band for lumbago into the cylindrical part. Therefore, when this slit is provided at a position extremely close to one of both ends of the cylindrical part front portion, the easiness of work is almost equivalent to that in the conventional case where the protection band for lumbago is inserted from one of both ends of the cylindrical part, so that the effect of providing the slit in the cylindrical part front portion extremely decreases. For the above-described reason, it is preferable that the slit 25 be disposed in the front portion of the cylindrical part and at the central part of the cylindrical part in the longitudinal direction, and the position of the slit 25 disposed in the cylindrical part may be a position closer to one end side of the cylindrical part with respect to the central part of the cylindrical part in the longitudinal direction as long as the workability for attaching the protection band for lumbago to the bottom wear is not considerably adversely affected. The substantially central part of the cylindrical part in the longitudinal direction is a part of the cylindrical part located at the center when the cylindrical part is divided into three equal parts in the longitudinal direction.

For the above-described reason, only one slit is provided at the substantially central part of the cylindrical part front portion in the bottom wear of the first embodiment.

Further, the cylindrical part is provided with only one slit that satisfies the following conditions (1) and (2) :
(1) the slit is provided in such a manner that the longitudinal direction of the slit is substantially orthogonal to the waist-encircling direction; and
(2) the ratio of the width (W2) of the slit in the longitudinal direction to the width (W1) of the cylindrical part in a direction orthogonal to the waist-encircling direction (W2/W1) is 0.70 to 1.00.

The slit is provided in such a manner that the longitudinal direction of the slit is substantially orthogonal to the waist-encircling direction. The phrase "the longitudinal direction is substantially orthogonal to the waist-encircling direction" means that the acute angle formed by the longitudinal direction and a direction orthogonal to the waist-encircling direction is 0 to 15 degrees. By disposing the slit in this way, the direction in the protection band for lumbago passes into the cylindrical part is substantially parallel to the longitudinal direction of the cylindrical part, so that work for attaching the protection band for lumbago becomes easy. Here, the shape of the slit is not particularly limited, and examples thereof include a substantially elliptical slit as shown in Figs. 1 and 24. In addition, this slit may be one obtained merely by making a notch at the center of a planar material in the front portion of the cylindrical part, or one in which the edge of the opening of the slit is sandwiched between cloths to surround the opening, followed by stitching. Among these forms, the slit is preferably one in which the edge of the opening of the slit is reinforced such as a slit in which the edge of the opening of the slit is sandwiched between cloths to surround the opening, followed by stitching. This is because a slit in which the edge of the opening of the slit is reinforced can be inhibited from being damaged by wearing of the edge of the opening of the slit due to friction between the edge of the opening of the slit and the protection band for lumbago. The slit is particularly preferably one in which the entire edge of the opening of the slit is reinforced. Further, the cloth used for surrounding the opening of the slit is preferably a stretchable cloth for making it easy to put in and take out the protection band for lumbago, and examples of the material for obtaining the stretchable cloth include those similar to the material of a planar material of the cylindrical part front portion as described later.

Further, as shown in Fig. 1, the ratio of W2 to W1 (W2/W1) is 0.70 to 1.00 where the width of the cylindrical part is W1 and the width of the slit in the longitudinal direction is W2. Here, as a precondition, from the viewpoint of exhibiting excellent retainability of protection band for lumbago by the bottom wear, the width of the waist portion protecting portion of the protection band for lumbago in a direction substantially parallel to the wearer's stature height direction is preferably as close as possible to the width of the cylindrical part in a direction substantially parallel to the wearer's stature height direction. The direction substantially parallel to the wearer's stature height direction is also a direction substantially parallel to the vertical direction, and is a direction parallel to a direction I as described later. Hereinafter, the direction is sometimes referred to as a substantially vertical direction.

Therefore, for exhibiting excellent retainability of the protection band for lumbago by the bottom wear, the wearer of the bottom wear typically employs, as a protection band for lumbago to be attached to the bottom wearer, a protection band for lumbago in which the width of the waist portion protecting portion of the protection band for lumbago in the substantially vertical direction is close to the width of the cylindrical part of the bottom wear in the substantially vertical direction. In this case, since the ratio of W2 to W1 (W2/W1) is 0.70 or more, it is easy to perform work in which the protection band for lumbago is put into the cylindrical part and taken out from the cylindrical part while the form stability of the cylindrical part front portion is maintained, and a protection band for lumbago including a waist portion protecting portion sufficiently large in width in the substantially vertical direction of the waist portion protecting portion can be employed as a protection band for lumbago to be attached to a bottom wear (as described in detail later, a protection band for lumbago including a waist portion protecting portion sufficiently large in width with the width being 5 to 20 cm in the substantially vertical direction tends to exhibit excellent waist protection performance). In addition, since the ratio of W2 to W1 (W2/W1) is 1.00 or less, deformation of the cylindrical part can be minimized when the protection band for lumbago is put into and taken out from the cylindrical part, so that the form stability of the entire cylindrical part can be maintained. In view of these points, the ratio of W2 to W1 (W2/W1) is set within the above-described range for securing a sufficient slit width for inserting and removing the protection band for lumbago while maintaining the form stability of the cylindrical part front portion. From the above-described viewpoint, the ratio of W2 to W1 (W2/W1) is more preferably 0.80 or more, and more preferably 0.98 or less. W1 refers to a value of the maximum width among widths of the cylindrical part in a direction orthogonal to the waist-encircling direction in the cylindrical part provided with the slit, and W2 refers to a value of the maximum width among widths of the slit in the longitudinal direction.

When W3 is a width of the slit in a direction orthogonal to the longitudinal direction, W3 is preferably 0.5 cm or more and 3 cm or less. When W3 is 0.5 cm or more, it is easy to attach the protection band for lumbago to the cylindrical part even if the overall rigidity of the protection band for lumbago is high, so that the protection band for lumbago is difficult to greatly bend. Specifically, when one end of the protection band for lumbago is caused to pass into the cylindrical part through the slit provided in the cylindrical part, and the other end of the protection band for lumbago is inserted into the slit, it is necessary to greatly bend a portion of the protection band for lumbago which extends from the cylindrical part, but the degree of bending of the part of the protection band for lumbago which extends from the cylindrical part decreases as W3 increases, and as a result, it is easy to attach the protection band for lumbago to the cylindrical part. On the other hand, when W3 is 3 cm or less, fixation of the protection band for lumbago to the bottom wear by the cylindrical part is further enhanced. Specifically, as W3 decreases, the portion of the protection band for lumbago which is attached to the bottom wear by the slit provided in the cylindrical part and is not covered with the cylindrical part becomes smaller, so that the protection band for lumbago is more firmly fixed to the bottom wear by the cylindrical part. W3 refers to a value of the maximum width among widths of the slit in a direction orthogonal to the longitudinal direction.

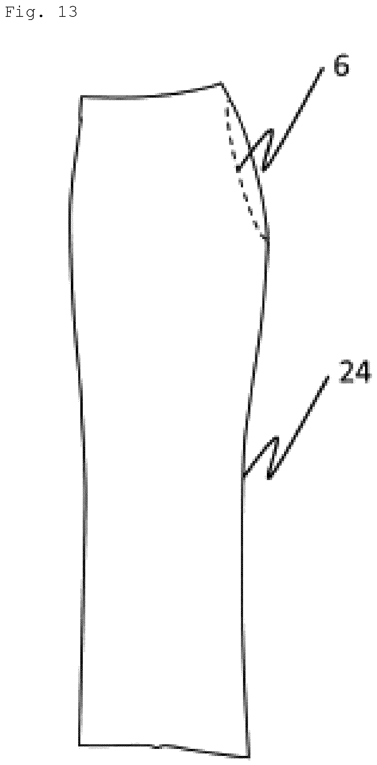

The cloth of bottom wear in the bottom wear according to the first embodiment will now be described. In the bottom wear of this embodiment, the cloth of bottom wear is a cloth other than the planar material forming the cylindrical part front portion, among members forming the bottom wear. Fig. 13 is an external view of the bottom wear shown in Fig. 1. In Fig. 13, a planar material forming the cylindrical part front portion 6 is shown by a broken line, which is an invisible part, and a cloth of bottom wear 24 is shown by a solid line.

Fig. 12 is a sectional view taken along line E-E' of the bottom wear shown in Fig. 2. In the embodiment of the bottom wear of the present invention shown in Fig. 1, a cylindrical part including the bottom wear cloth 5 on the back portion in the waist-encircling section, and a planar material forming the cylindrical part front portion 6 is formed in a back portion 18 in the waist-encircling section. In addition, a space in the cylindrical part, i.e. a space between the bottom wear cloth 5 on the back portion in the waist-encircling section and the cylindrical part front portion 6 is a space 21 for placing a protection band for lumbago, and in a state of a bottom wear with a band, to which a band is attached, a waist portion protecting portion of the protection band for lumbago is stored in the space 21 for placing a protection band for lumbago.

Next, Fig. 4 is a front view showing one surface of one example of a protection band for lumbago which can be attached to any of the bottom wears of the first and second embodiments. Fig. 5 is a rear view of the protection band for lumbago. The protection band for lumbago 9 includes a waist portion protecting portion 10, a belt portion 11 disposed on each of the left and the right of the waist portion protecting portion 10, a first fixing member 12 disposed at one end portion of the protection band for lumbago, a second fixing member 13 disposed at the other end portion of the protection band for lumbago, and a bone 14 disposed at the center of the waist portion protecting portion 10.

The waist portion protecting portion 10 of the protection band for lumbago 9 shown in Figs. 4 and 5 is in the form of a band having a large width. The large width is suitable for disposing the bone. Unlike the illustrated waist portion protecting portion, the waist portion protecting portion may have a width equal to or smaller than the width of the belt portion. In addition, the width (back width) of the waist portion protecting portion in a stature direction of a wearer is preferably 5 to 20 cm. When the back width is 5 cm or more, a fastening pressure on a waist portion of the wearer is concentrated on a narrow width portion and becomes relatively high, thereby enabling a blood vessel to be inhibited from being excessively compressed, and further, in positioning of the waist portion protecting portion, a burden for positioning the waist portion protecting portion on an upper part of a pelvis can be reduced. When the back width is 20 cm or less, oppressive heat can be reduced at the time of wearing the bottom wear for long hours. In consideration of these points mentioned above, in order to allow the bottom wear to be used for long hours and secure workability while minimizing a limitation in a range of motion, it is preferable to set the back width of the waist portion protecting portion to be within the mentioned range. In addition, from the viewpoint mentioned above, it is more preferable that the back width be 8 cm or more, and the width be 15 cm or less.

The waist portion protecting portion may be made of a material being the same as or different from that of the belt portion. Fabric, synthetic leather or the like can be used as the material of the waist portion protecting portion and the belt portion. The material is preferably fabric because the protection band for lumbago can be made washable. As the material of the above-mentioned fabric, synthetic fibers such as polyethylene terephthalate fiber and polyamide fiber, natural fibers such as cotton fiber, regenerated fibers such as rayon fiber, and the like can be used. In addition, as the material of the above-mentioned fabric, a plurality of the above-described fibers can be used.

Further, it is preferable that the waist portion protecting portion be provided with bones for stabilizing a position of a spinal column. Here, the material of the bone is preferably a metal, a resin or the like, which has a relatively high bending stress. Examples of the shape include a spiral shape, a strip bone shape whose longitudinal direction is the stature height direction of the wearer, and a plate shape that covers the waist portion protecting portion. In addition, the strip bone shape and the plate bone shape may be used in combination.

In addition, the waist portion protecting portion may be provided with a slip prevention member (not shown).

When in a state in which the protection band for lumbago is attached to the bottom wear of the present invention, the first fixing member and the second fixing member of the protection band for lumbago are joined together in front of the wearer, the pressure on the waist portion of the wearer can be increased. It is preferable that at least one of the first fixing member and the second fixing member is long in the longitudinal direction of the protection band for lumbago because the adjustable range of the pressure on the waist portion of the wearer can be expanded. Further, for the purpose of expanding the adjustable range of the pressure on the waist of the wearer, an auxiliary belt composed of band-shaped flat rubber or the like may be provided.

Here, it is preferable to use a hook-and-loop fastener as a material for the first fixing member and the second fixing member of the protection band for lumbago so that the first fixing member and the second fixing member are mutually locked. In this case, the type of hook-and-loop fastener is not particularly limited, but when considering that pilling occurs in the cloth of bottom wear due to damage to the cloth of bottom wear, it is preferable to use a hook-and-loop fastener of mushroom type in which the tip portion of a protrusion portion of a male surface is rounded.

Fig. 6 is a view showing a state in which the protection band for lumbago shown in Fig. 4 is attached to the bottom wear in Fig. 1, where the bottom wear is seen from the front and above. Fig. 7 is a sectional view taken along line B-B' of the bottom wear and protection band for lumbago attached thereto in Fig. 6. In this embodiment, the protection band for lumbago 9 is inserted and disposed inside the cylindrical part 3 in such a manner that the longitudinal direction of the protection band for lumbago 9 is substantially parallel to the axis direction of the cylindrical part 3, and a surface of the protection band for lumbago, which is provided with a bone (hereinafter, the surface of the protection band for lumbago is sometimes referred to as a "surface A of protection band for lumbago"), is on the wearer side at the time of wearing the bottom wear. The right end portion of the protection band for lumbago is protruded from the cylindrical part outside the cylindrical part on the right side of the cylindrical part 3 as shown in Fig. 6. The left end portion of the protection band for lumbago is protruded from the cylindrical part outside the cylindrical part on the left side of the cylindrical part 3. In addition, the waist portion protecting portion of the protection band for lumbago is covered with the cylindrical part, and the inner surface of the cylindrical part rear portion is in contact with a surface on the side opposite to the surface A of protection band for lumbago (hereinafter, the surface of the protection band for lumbago is sometimes referred to as a "surface B of protection band for lumbago"). That is, the surface B of protection band for lumbago is in contact with the inner surface of the bottom wear cloth on the back portion in the waist-encircling section of the cloth of bottom wear.

See Fig. 3. In the bottom wear of the first embodiment, the bottom wear cloth 5 on the back portion in the waist-encircling section corresponds to the cylindrical part rear portion. Thus, the static friction coefficient of an inner surface 22 is preferably 0.2 to 1.0.

In addition, the planar material forming the cylindrical part front portion 6 in Fig. 3 has a stress of 10 N/25 mm or less at 10% elongation in a transverse direction (hereinafter, sometimes referred to as a direction I). Here, the direction I is also a direction substantially parallel to the wearer's stature height direction at the time of wearing the bottom wear.

Here, the material of the planar material is not particularly limited, but a fabric is preferable because it is excellent in versatility. In particular, a woven fabric or a knitted fabric is more preferable. Examples of the fiber used for the fabric may include synthetic fibers and natural fibers, and a plurality of fibers may be used. When considering washing resistance and versatility of original yarn/weaving and knitting design, it is preferable that synthetic fiber such as polyester fiber or nylon fiber be used alone, or synthetic fiber such as polyester fiber or nylon fiber and at least one selected from the group consisting of polyurethane fiber, rayon fiber, acrylic fiber and cotton fiber be used in combination.

As described above, it is preferable that the planar material of the cylindrical part front portion (symbol 6 in Fig. 7) have a stress of 10 N/25 mm or less at 10% elongation in a transverse direction, i.e., a direction I. A fabric that can be used for achieving the above-mentioned stress is a woven fabric using a stretchable yarn as each of warp and weft yarns or as one of warp and weft yarns, or a knitted fabric more extensible as compared to a woven fabric. The knitted fabric can be obtained by appropriately adjusting the blending ratio of a stretchable yarn and a non-stretchable yarn. Specific examples of the fabric having a stress of 10 N/25 mm or less at 10% elongation in the direction I include the following fabrics.
(I) Woven fabrics composed of fiber obtained by mixing elastic fiber (such as polyurethane fiber) with natural fiber such as silk or wool fiber, regenerated fiber such as rayon fiber, synthetic fiber such as acrylic fiber or polyester fibers, or the like.
(II) Knitted fabrics composed of fiber obtained by mixing elastic fiber (such as polyurethane fiber) with natural fiber such as silk or wool fiber, regenerated fiber such as rayon fiber, synthetic fiber such as acrylic fiber or polyester fibers, or the like.
(III) Woven fabrics in which elastic fiber is not used, but multifilaments of composite fiber obtained by bonding two types of polyester resins, one of which is a polyester resin mainly composed of polytrimethylene terephthalate (PTT) and the other of which is a polyester resin mainly composed of polyethylene terephthalate, side by side along the fiber length are used as at least one of a warp yarn and a weft yarn.
(IV) Knitted fabrics in which elastic fiber is not used, but multifilaments of composite fiber obtained by bonding two types of polyester resins, one of which is a polyester resin mainly composed of polytrimethylene terephthalate (PTT) and the other of which is a polyester resin mainly composed of polyethylene terephthalate, side by side along the fiber length are used.
(V) Power net.

Here, the power net refers to a fine net-like cloth produced from nylon fiber or the like and elastic fiber (e.g. polyurethane fiber). The power net may be a relatively thin cloth having an areal weight of about 100 g/m², and the cylindrical part front portion 6 (i.e. planar material) is preferably a power net because it is excellent in elongation and kickback performance, and capable of making the cylindrical part compact while securing the function of the cylindrical part provided on the back portion of the bottom wear.

As described above, the static friction coefficient of the inner surface 22 of the cylindrical part rear portion (the bottom wear cloth 5 on the back portion in the waist-encircling section in Fig. 7) is preferably 0.2 or more and 1.0 or less. The reason why this static friction coefficient is selected will be described later. Here, examples of the means for setting the static friction coefficient of the inner surface 22 of the cylindrical part rear portion to 0.2 to 1.0 include the following.

The cloth of bottom wear is formed into a woven fabric or a knitted fabric, and it is possible to adjust the static friction coefficient according to the irregular state of the surface of this cloth and the kind of yarn forming the cloth. For the woven fabric, the static friction coefficient is small in the case of a plain weave having a small number of irregularities on the surface, and conversely, the static friction coefficient is large in the case of a twill weave in which oblique ridges appear on the woven fabric surface, and a satin weave in which oblique ridges do not appear, but tissue points are arranged at regular intervals. In addition, the static friction coefficient is generally increased by providing a pile on the surface of the cloth or raising the surface. For the knitted fabric, weft knitting or warp knitting may be performed, and the static friction coefficient can be adjusted according to the density of the knitted fabric. However, warp knitting is preferable because even when the stitch is cut, a run hardly occurs, resulting in enhancement of durability. The static friction coefficient can also be adjusted according to the shape of the yarn to be used for the woven or knitted fabric. For example, since the irregularities on the fiber surface of a filament yarn is smaller as compared to a spun yarn, the static friction coefficient of a cloth using the filament yarn can be reduced. In addition, the static friction coefficient can also be adjusted according to the material of the yarn. For example, polytetrafluoroethylene fiber (PTFE fiber) itself has a low static friction coefficient, and conversely, rubber or a polyurethane yarn itself has a high static friction coefficient. By using such a yarn for a part or the whole of the cloth, the static friction coefficient as a cloth can be adjusted. Further, the static friction coefficients of the surface and the back surface of the cloth can be set to different values. When on one surface of a double-side knitted structure (tricot), a knit loop is formed with only an elastic yarn to form a warp knitted fabric, and on the other surface, a knit loop is formed by use of an elastic yarn and an inelastic yarn in combination to form a warp knitted fabric, a high static friction coefficient is developed for the surface on which the knit loop is formed with only an elastic yarn.

Further, the surface 27 of a portion of the cylindrical part front portion which is far from the human body (i.e. a surface of the front portion of the cylindrical part on a side opposite to the surface on the wearer side) is preferably 0.2 to 1.0. In addition, as a method for adjusting the static friction coefficient, the static friction coefficient is adjusted according to irregularities on the surface of the woven or knitted structure, and the shape and type of a yarn as in the method for adjusting the static friction coefficient of the inner surface of the bottom wear cloth 5 on the back portion in the waist-encircling section. Since the static friction coefficient of the surface of the cylindrical part front portion which is far from the human body is 0.2 to 1.0, the effect of simplifying the work for inserting the protection band for lumbago in the bottom wear of the first embodiment becomes more remarkable.

In addition, the static friction coefficient of an outer surface 23 of the cylindrical part front portion (i.e., a surface of the cylindrical part, which is close to the human body) is preferably 0.2 to 1.0. In addition, as a method for adjusting the static friction coefficient, the static friction coefficient is adjusted according to irregularities on the surface of the woven or knitted structure, and the shape and type of a yarn as in the method for adjusting the static friction coefficient of the inner surface of the bottom wear cloth 5 on the back portion in the waist-encircling section. Since the static friction coefficient of the outer surface of the cylindrical part front portion is 0.2 to 0.1, an underwear worn by a wearer can be prevented from being slid in a wearer's stature direction with respect to a waist portion of the wearer as the wearer moves, so that the wear comfort for the wearer's can be improved.

Subsequently, a bottom wear having a cylindrical part according to the second embodiment will be described below.

The cylindrical part according to the second embodiment includes a planar material different from a cloth of bottom wear.

Fig. 8 is a view of the bottom wear according to the second embodiment where the bottom wear is seen from the front and above. Fig. 9 is a sectional view taken along line C-C' of the bottom wear shown in Fig. 8. In this embodiment, as shown in Fig. 8, the bottom wear 1 has a waist-encircling section 2, and a cylindrical part 3 on the back potion of the waist-encircling section 2. Further, the axis direction of the cylindrical part 3 is substantially parallel to a waist-encircling direction of the waist-encircling section. The cylindrical part according to the second embodiment includes a planar material different from the cloth of bottom wear, and the cylindrical part includes a cylindrical part front portion 15 and a cylindrical part rear portion 15' as shown in Fig. 9. The upper end and the lower end of the cylindrical part are connected to the bottom wear cloth on the back portion in the waist-encircling section of the cloth of bottom wear by a locking portion 16 and a locking portion 17, respectively.

Further, only one slit 26 is disposed at the central part of the cylindrical part front portion 15 shown in Figs. 8 and 9, and the slit 26 is provided in such a manner that the longitudinal direction is substantially orthogonal to the waist-encircling direction. If there is no slit 26, the cylindrical part will be as shown in Fig. 22. In the structure of such a cylindrical part, when a protection band for lumbago is attached, one end of the band is inserted through one opening of the cylindrical part, and caused to pass toward the other opening of the cylindrical part, so that the band is attached. In such an attachment method, the band may be stuck in the middle, or bent within the cylindrical part. In this case, the worker should insert his or her hand to the inside of the cylindrical part through the other opening of the cylindrical part to pull out the band. However, even when a hand is inserted through the other opening of the cylindrical part to pull out the band as mentioned above, it tends to be difficult to insert the hand to a position at which the band is stuck if the band passage distance such as a distance from one opening to the other opening of the cylindrical part is long. Due to the above-mentioned various problems, a bottom wear having a configuration, which cannot choose but employ a method in which a band is inserted through an opening disposed at one of both ends of the cylindrical part, has the problem that it takes much time for the wearer to complete attachment of the protection band for lumbago to the cylindrical part. However, by providing the slit 26, the protection band for lumbago can be easily attached to the bottom wear in accordance with a procedure as shown in Figs. 23, 24 and 25. Specifically, first, one of the left and right ends of the protection band for lumbago with respect to the center of the back portion is caused to pass into the cylindrical part (Fig. 24), and the other end is caused to pass into the cylindrical part on the opposite side (Fig. 25), whereby the protection band for lumbago can be attached, so that it is possible to considerably reduce the time for inserting protection band for lumbago. As shown in Fig. 8, the cylindrical part is provided with only one slit 26. The reason for limiting the number of slits 26 to one is as follows. The slit in the bottom wear of the second embodiment is disposed for the purpose of facilitating work for inserting the protection band for lumbago through the slit and inserting the protection band for lumbago into the cylindrical part. Therefore, if the number of slits is two or more (for example slits A and B), the tip of the protection band for lumbago is caught in the slit B of the protection band for lumbago at the time of inserting the protection band for lumbago into the cylindrical part through the slit A, so that it is difficult for the band to smoothly enter through the slit A, resulting in rather expansion of time-consuming work. Thus, it tends to be impossible to obtain an effect of providing slits in the cylindrical part front portion. Further, as the number of slits increases, the time-consuming work expands as described above, so that the effect of providing slits in the cylindrical part front portion decreases. For the reason described above, the number of slits provided in the cylindrical part front portion is limited to one.

Further, the slit is provided in the front portion of the cylindrical part and in the substantially central part of the cylindrical part in the longitudinal direction. The reason why the position at which the slit is provided is set to the cylindrical part front portion and the substantially central part of the cylindrical part in the longitudinal direction is as follows. First, since the slit is provided at the cylindrical part front portion, the protection band for lumbago can be attached to and detached from the cylindrical part while the bottom wear is worn, and thus attachment/detachment of the protection band for lumbago is easier as compared to a case where the slit is provided in the rear portion of the cylindrical part of the protection band for lumbago. As described above, the slit in the bottom wear of the second embodiment is disposed for the purpose of facilitating work for inserting the protection band for lumbago through the slit and inserting the protection band for lumbago into the cylindrical part. Therefore, when this slit is provided at a position extremely close to one of both ends of the cylindrical part front portion, the easiness of work is almost equivalent to that in the conventional case where the protection band for lumbago is inserted from one of both ends of the cylindrical part, so that the effect of providing the slit in the cylindrical part front portion extremely decreases. For the above-described reason, it is preferable that the slit 26 be disposed in the front portion of the cylindrical part and at the central part of the cylindrical part in the longitudinal direction, and the position of the slit 26 disposed in the cylindrical part may be a position closer to one end side of the cylindrical part with respect to the central part of the cylindrical part in the longitudinal direction as long as the workability for attaching the protection band for lumbago to the bottom wear is not considerably adversely affected. The substantially central part of the cylindrical part in the longitudinal direction is a part of the cylindrical part located at the center when the cylindrical part is divided into three equal parts in the longitudinal direction.

For the above-described reason, only one slit is provided at the substantially central part of the cylindrical part front portion in the bottom wear of the second embodiment.

Further, the cylindrical part is provided with only one slit that satisfies the following conditions (1) and (2) :
(1) the slit is provided in such a manner that the longitudinal direction of the slit is substantially orthogonal to the waist-encircling direction; and
(2) the ratio of the width (W2) of the slit in the longitudinal direction to the width (W1) of the cylindrical part in a direction orthogonal to the waist-encircling direction (W2/W1) is 0.70 to 1.00.

The slit is provided in such a manner that the longitudinal direction of the slit is substantially orthogonal to the waist-encircling direction. The phrase "the longitudinal direction is substantially orthogonal to the waist-encircling direction" means that the acute angle formed by the longitudinal direction and a direction orthogonal to the waist-encircling direction is 0 to 15 degrees. By disposing the slit in this way, the direction in the protection band for lumbago passes into the cylindrical part is substantially parallel to the longitudinal direction of the cylindrical part, so that work for attaching the protection band for lumbago becomes easy. Here, the shape of the slit is not particularly limited, and examples thereof include a substantially elliptical slit as shown in Figs. 8 and 24. In addition, this slit may be one obtained merely by making a notch at the center of a planar material in the front portion of the cylindrical part, or one in which the edge of the opening of the slit is sandwiched between cloths, followed by stitching. Since the protection band for lumbago is inserted and removed through this slit many times, it is more preferable to sandwich the edge of the opening of the slit by a cloth and stitch to surround the edge from the viewpoint of enhancing the durability of the slit. Further, the cloth used for surrounding the opening of the slit is preferably a stretchable cloth for making it easy to put in and take out the protection band for lumbago, and examples of the material for obtaining the stretchable cloth include those similar to the material of the planar material of the cylindrical part front portion as described in the first embodiment.

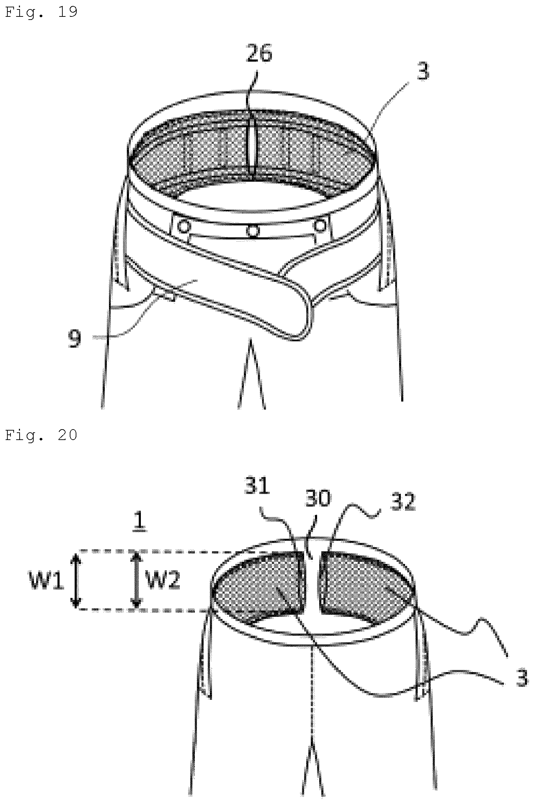

Further, it is preferable that the slit be present only in the front portion of the cylindrical part. Presence of the slit only in the front portion of the cylindrical part suppresses expansion of time-consuming work for inserting the protection band for lumbago, such that at the time of inserting the protection band for lumbago through the slit, the protection band for lumbago accidentally enters between the cylindrical part rear portion and the cloth of bottom wear, and the protection band for lumbago should be pulled out and inserted into the slit. Thus, it is possible to smoothly insert the protection band for lumbago. For the above reasons, presence of the slit only in the front portion of the cylindrical part is preferable because presence of the slit only in the front portion of the cylindrical part simplifies work for attaching the protection band for lumbago as compared to a case where the slit is also present in the rear portion of the cylindrical part as compared to Fig. 20.

Further, as shown in Fig. 8, the ratio of W2 to W1 (W2/W1) is 0.70 to 1.00 where the width of the cylindrical part is W1 and the width of the slit in the longitudinal direction is W2 in the bottom wear of the second embodiment. Here, as a precondition, from the viewpoint of exhibiting excellent retainability of protection band for lumbago by the bottom wear, the width of the waist portion protecting portion of the protection band for lumbago in a direction substantially parallel to the wearer's stature height direction (i.e. a substantially parallel to the vertical direction, and a direction parallel to the direction I, and is sometimes referred to as a substantially vertical direction) is preferably as close as possible to the width of the cylindrical part in a direction substantially parallel to the wearer's stature height direction. Therefore, for exhibiting excellent retainability of the protection band for lumbago by the bottom wear, the wearer of the bottom wear typically employs, as a protection band for lumbago to be attached to the bottom wearer, a protection band for lumbago in which the width of the waist portion protecting portion of the protection band for lumbago in the substantially vertical direction is close to the width of the cylindrical part of the bottom wear in the substantially vertical direction. In this case, since the ratio of W2 to W1 (W2/W1) is 0.70 or more, it is easy to perform work in which the protection band for lumbago is put into the cylindrical part and taken out from the cylindrical part while the form stability of the cylindrical part front portion is maintained, and a protection band for lumbago including a waist portion protecting portion sufficiently large in width in the substantially vertical direction of the waist portion protecting portion can be employed as a protection band for lumbago to be attached to the bottom wear. As described above, a protection band for lumbago including a waist portion protecting portion having a sufficiently large width of 5 to 20 cm in the substantially vertical direction tends to exhibit excellent waist protecting performance. In addition, since the ratio of W2 to W1 (W2/W1) is 1.00 or less, deformation of the cylindrical part can be minimized when the protection band for lumbago is put into and taken out from the cylindrical part, so that the form stability of the entire cylindrical part can be maintained. In view of these points, the ratio of W2 to W1 (W2/W1) is set within the above-described range for securing a sufficient slit width for inserting and removing the protection band for lumbago while maintaining the form stability of the cylindrical part front portion. From the above-described viewpoint, the ratio of W2 to W1 (W2/W1) is more preferably 0.80 or more, and more preferably 0.98 or less. W1 refers to a value of the maximum width among widths of the cylindrical part in a direction orthogonal to the waist-encircling direction in the cylindrical part provided with the slit, and W2 refers to a width of the slit in the longitudinal direction.

When W3 is a width of the slit in a direction orthogonal to the longitudinal direction, W3 is preferably 0.5 cm or more and 3 cm or less. When W3 is 0.5 cm or more, it is easy to attach the protection band for lumbago to the cylindrical part even if the overall rigidity of the protection band for lumbago is high, so that the protection band for lumbago is difficult to greatly bend. Specifically, when one end of the protection band for lumbago is caused to pass into the cylindrical part through the slit provided in the cylindrical part, and the other end of the protection band for lumbago is inserted into the slit, it is necessary to greatly bend a portion of the protection band for lumbago which extends from the cylindrical part, but the degree of bending of the part of the protection band for lumbago which extends from the cylindrical part decreases as W3 increases, and as a result, it is easy to attach the protection band for lumbago to the cylindrical part. On the other hand, when W3 is 3 cm or less, fixation of the protection band for lumbago to the bottom wear by the cylindrical part is further enhanced. Specifically, as W3 decreases, the portion of the protection band for lumbago which is attached to the bottom wear by the slit provided in the cylindrical part and is not covered with the cylindrical part becomes smaller, so that the protection band for lumbago is more firmly fixed to the bottom wear by the cylindrical part. W3 refers to a value of the maximum width among widths of the slit in a direction orthogonal to the longitudinal direction.

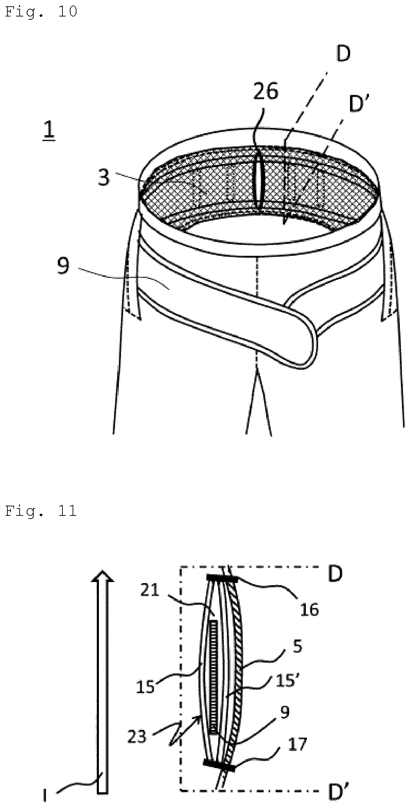

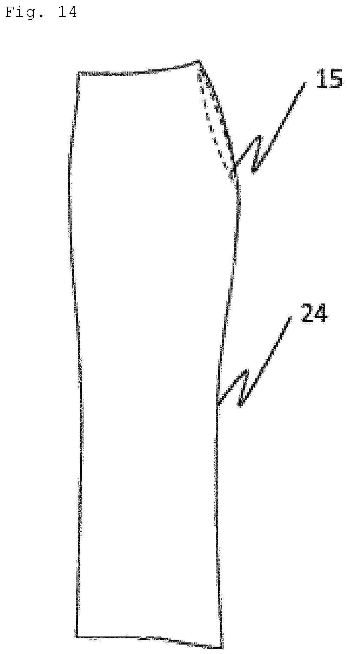

Fig. 14 is an appearance view of the bottom wear of the second embodiment as shown in Fig. 8. In Fig. 14, the fabric 15 having a cylindrical shape is shown by a broken line, which is an invisible part, and the cloth of bottom wear 24 is shown by a solid line. Fig. 10 is a view showing a state in which the protection band for lumbago shown in Figs. 4 and 5 is attached to the bottom wear shown in Fig. 8 as the second embodiment, where the bottom wear is seen from the front and above. Fig. 11 is a sectional view taken along line D-D' of the bottom wear shown in Fig. 10, to which a protection band for lumbago is attached.

See Fig. 10. In the second embodiment, the protection band for lumbago 9 is inserted and disposed inside the cylindrical part 3. The longitudinal direction of the protection band for lumbago 9 is substantially parallel to the axis direction of the cylindrical part 3, and the surface A of the protection band for lumbago is on the wearer side at the time of wearing the bottom wear. The right end portion of the protection band for lumbago on the right side of the cylindrical part 3 is protruded from the cylindrical part, and the left end portion of the protection band for lumbago is protruded from the cylindrical part outside the cylindrical part on the left side of the cylindrical part. In addition, the waist portion protecting portion of the protection band for lumbago is covered with the cylindrical part, and the inner surface of the cylindrical part rear portion may be in contact with the surface B of the protection band for lumbago. That is, the surface B of protection band for lumbago may be in contact with a surface on a side opposite to a surface that is in contact with the inner surface of the bottom wear cloth on the back portion in the waist-encircling section of the cloth of bottom wear of a fabric 15 having a cylindrical shape.

In the bottom wear of the second embodiment, the static friction coefficient of the inner surface (surface denoted by symbol 22 in Fig. 9) of the cylindrical part rear portion is preferably 0.2 to 1.0. The reason why this static friction coefficient is selected is as described below. The cylindrical part front portion has a stress of preferably 10 N/25 mm or less at 10% elongation in a transverse direction (direction I).

In the bottom wear of the second embodiment, the material of the planar material forming the cylindrical part is not particularly limited, but is preferably a fabric. As materials having a static friction coefficient as described above and a stress at elongation as described above, those described in the first embodiment can be used. Examples of the method for forming the cylindrical part include a method in which a fabric is folded, and the upper parts are bonded; a method in which two or more fabrics are stitched together; and a method in which a circular knitted fabric is used as such to form a cylindrical shape. The material may be used as such to form the cylindrical part.

Further, the surface 27 of a portion of the cylindrical part front portion which is far from the human body (i.e., a surface of the front portion of the cylindrical part on a side opposite to the surface on the wearer side) is preferably 0.2 to 1.0. In addition, as a method for adjusting the static friction coefficient, the static friction coefficient is adjusted according to irregularities on the surface of the woven or knitted structure, and the shape and type of a yarn as in the method for adjusting the static friction coefficient of the inner surface of the bottom wear cloth 5 on the back portion in the waist-encircling section in the first embodiment. Since the static friction coefficient of the surface of the cylindrical part front portion which is far from the human body is 0.2 to 1.0, the effect of simplifying the work for inserting the protection band for lumbago in the bottom wear of the second embodiment becomes more remarkable.

In addition, the static friction coefficient of an outer surface 23 of the cylindrical part front portion (i.e., a surface of the cylindrical part, which is close to the human body) is preferably 0.2 to 1.0. In addition, as a method for adjusting the static friction coefficient, the static friction coefficient is adjusted according to irregularities on the surface of the woven or knitted structure, and the shape and type of a yarn as in the method for adjusting the static friction coefficient of the inner surface of the bottom wear cloth 5 on the back portion in the waist-encircling section in the first embodiment. Since the static friction coefficient of the outer surface of the cylindrical part front portion is 0.2 to 0.1, an underwear worn by a wearer can be prevented from being slid in a wearer's stature direction with respect to a waist portion of the wearer as the wearer moves, so that the wear comfort for the wearer's can be improved.

The cylindrical part formed by the cylindrical part front portion 15 and rear portion 15' is continuously or intermittently connected to the bottom wear cloth 5 at the upper end by the locking portion 16 in the waist-encircling direction. Preferably, the upper end of the cylindrical part is connected to the waist-encircling section because it is possible to suppress a situation in which the toe or the like of a wearer is caught between the cylindrical part and the bottom wear cloth 5 at the time of wearing the bottom wear of the present embodiment.

It is to be noted that the present invention is not limited to the configuration shown in Fig. 9. A portion slightly below the upper end of the planar material (a part situated below the upper end of the planar material) may be connected, i.e., the upper end of the planar material may be protruded above the locking portion.

In addition, in Fig. 9, the lower end of the cylindrical part is intermittently connected in a waist-encircling direction to the bottom wear cloth 5 on the back portion in the waist-encircling section by a locking portion 17. The reason why it is preferable that the planar material be connected at the lower end is as follows.

The lower end portion of the cylindrical part can be fixed so as to extend along the inner surface of the cloth of bottom wear. As a result, a protection band for lumbago is neatly inserted into the cylindrical part. In addition, it is possible to suppress uncontrollable movement of the lower end portion of the cylindrical part inside the bottom wear. It is possible to suppress bending of the cylindrical part inside the bottom wear, and therefore a wearer can insert a protection band for lumbago into the cylindrical part while wearing the bottom wear.

The present invention is not limited to the configuration shown in Fig. 9. A portion slightly above the lower end of the planar material (a part situated above the lower end of the planar material) may be connected, i.e., the lower end of a fabric may be protruded below the locking portion.

In the bottom wear configured to have a cross-section as in Fig. 9 in the present invention, the cylindrical part is connected to the bottom wear cloth on the back portion by the locking portion 16 and the locking portion 17. However, the upper end or the lower end of the fabric may be continuously connected to the bottom wear cloth.

Here, examples of the intermittent or continuous locking portion include the means described in the first embodiment.

Further, in the second embodiment as well as the first embodiment, only one slit is disposed at the central part of the cylindrical part front portion, and the slit is provided in such a manner that the longitudinal direction is orthogonal to the waist-encircling direction. By providing the slit in this way, the direction in which the protection band for lumbago passes into the cylindrical part is substantially parallel to the longitudinal direction of the cylindrical part, so that work for attaching the protection band for lumbago to the cylindrical part becomes easy. Here, the shape of the slit is not particularly limited, and examples thereof include a substantially elliptical slit as shown in Figs. 1 and 8. In addition, this slit may be one obtained merely by making a notch at the center of a planar material in the front portion of the cylindrical part, or one in which the edge of the opening of the slit is sandwiched between cloths, followed by stitching. Since the protection band for lumbago is inserted and removed through this slit many times, it is more preferable to sandwich the edge of the opening of the slit by a cloth and stitch to surround the edge from the viewpoint of enhancing the durability of the slit. Further, the cloth used for surrounding the opening of the slit is preferably a stretchable cloth for making it easy to put in and take out the protection band for lumbago, and examples of the material for obtaining the stretchable cloth include those similar to the material of a planar material of the cylindrical part front portion as described above.

As shown in Figs. 1 and 8, only one slit is provided in the front portion of the cylindrical part and in the substantially central part of the cylindrical part in the longitudinal direction. The reason for limiting the number of slits to one is as follows. The slit in the bottom wear of the present embodiment is disposed for the purpose of facilitating work for inserting the protection band for lumbago through the slit and inserting the protection band for lumbago into the cylindrical part. Therefore, if the number of slits is two or more (for example slits A and B), the tip of the protection band for lumbago is caught in the slit B of the protection band for lumbago at the time of inserting the protection band for lumbago into the cylindrical part through the slit A, so that it is difficult for the band to smoothly enter through the slit A, resulting in rather expansion of time-consuming work. Thus, it tends to be impossible to obtain an effect of providing slits in the cylindrical part front portion. Further, as the number of slits increases, the time-consuming work expands as described above, so that the effect of providing slits in the cylindrical part front portion decreases. For the reason described above, the number of slits provided in the cylindrical part front portion is limited to one.

Further, the reason why the position at which the slit is provided is set to the cylindrical part front portion and the central part of the cylindrical part in the longitudinal direction is as follows. As described above, the slit in the bottom wear of the present invention is disposed for the purpose of facilitating work for inserting the protection band for lumbago through the slit and inserting the protection band for lumbago into the cylindrical part. Therefore, when this slit is provided at a position extremely close to one of both ends of the cylindrical part front portion, the easiness of work is almost equivalent to that in the conventional case where the protection band for lumbago is inserted from one of both ends of the cylindrical part, so that the effect of providing the slit in the cylindrical part front portion extremely decreases. For the above-described reason, it is preferable that the slit be disposed in the front portion of the cylindrical part and at the central part of the cylindrical part in the longitudinal direction, and the position of the slit disposed in the cylindrical part may be a position closer to one end side of the cylindrical part with respect to the central part of the cylindrical part in the longitudinal direction as long as the workability for attaching the protection band for lumbago to the bottom wear is not considerably adversely affected. Here, the slit disposition position which does not significantly affect workability for attaching the protection band for lumbago to the bottom wear is referred to as a substantially central part of the cylindrical part in the longitudinal direction, and the substantially central part of the cylindrical part in the longitudinal direction refers to a part of a cylindrical part positioned at the center when the cylindrical part is divided into three equal parts in the longitudinal direction. For the above-described reason, only one slit is provided at the substantially central part of the cylindrical part front portion in the bottom wear of the present invention.

Further, in the second embodiment as well as the first embodiments, the ratio of W2 to W1 (W2/W1) is 0.70 to 1.00 where the width of the cylindrical part is W1 and the width of the slit in the longitudinal direction is W2. Here, as a precondition, from the viewpoint of exhibiting excellent retainability of protection band for lumbago by the bottom wear, the width of the waist portion protecting portion of the protection band for lumbago in a direction substantially parallel to the wearer's stature height direction (i.e. a substantially parallel to the vertical direction, and a direction parallel to the direction I) is preferably as close as possible to the width of the cylindrical part in a direction substantially parallel to the wearer's stature height direction. Therefore, for exhibiting excellent retainability of the protection band for lumbago by the bottom wear, the wearer of the bottom wear typically employs, as a protection band for lumbago to be attached to the bottom wearer, a protection band for lumbago in which the width of the waist portion protecting portion of the protection band for lumbago in the substantially vertical direction is close to the width of the cylindrical part of the bottom wear in the substantially vertical direction. In this case, since the ratio of W2 to W1 (W2/W1) is 0.70 or more, it is easy to perform work in which the protection band for lumbago is put into the cylindrical part and taken out from the cylindrical part while the form stability of the cylindrical part front portion is maintained, and a protection band for lumbago including a waist portion protecting portion sufficiently large in width in the substantially vertical direction of the waist portion protecting portion can be employed as a protection band for lumbago to be attached to the bottom wear (as described above, a protection band for lumbago including a waist portion protecting portion sufficiently large in width with the width being 5 to 20 cm in the substantially vertical direction tends to exhibit excellent waist protection performance). In addition, since the ratio of W2 to W1 (W2/W1) is 1.00 or less, deformation of the cylindrical part can be minimized when the protection band for lumbago is put into and taken out from the cylindrical part, so that the form stability of the entire cylindrical part can be maintained. In view of these points, the ratio of W2 to W1 (W2/W1) is set within the above-described range for securing a sufficient slit width for inserting and removing the protection band for lumbago while maintaining the form stability of the cylindrical part front portion. From the above-described viewpoint, the ratio of W2 to W1 (W2/W1) is more preferably 0.80 or more, and more preferably 0.98 or less. W1 refers to a value of the maximum width among widths of the cylindrical part in a direction direct to the waist-encircling direction in the front portion of the cylindrical part provided with the slit.

When W3 is a width of the slit in a direction orthogonal to the longitudinal direction, W3 is preferably 0.5 cm or more and 3 cm or less. When W3 is 0.5 cm or more, it is easy to attach the protection band for lumbago to the cylindrical part even if the overall rigidity of the protection band for lumbago is high, so that the protection band for lumbago is difficult to greatly bend. Specifically, when one end of the protection band for lumbago is caused to pass into the cylindrical part through the slit provided in the cylindrical part, and the other end of the protection band for lumbago is inserted into the slit, it is necessary to greatly bend a portion of the protection band for lumbago which extends from the cylindrical part, but the bending of the part of the protection band for lumbago which extends from the cylindrical part decreases as W3 increases, and as a result, it is easy to attach the protection band for lumbago to the cylindrical part. On the other hand, when W3 is 3 cm or less, fixation of the protection band for lumbago to the bottom wear by the cylindrical part is further enhanced. Specifically, as W3 decreases, the portion of the protection band for lumbago which is attached to the bottom wear by the slit provided in the cylindrical part and is not covered with the cylindrical part becomes smaller, so that the protection band for lumbago is more firmly fixed to the bottom wear by the cylindrical part. W3 refers to a value of the maximum width among widths of the slit in a direction orthogonal to the longitudinal direction.

In addition, in the second embodiment as well as the first embodiment, the static friction coefficient of the outer surface of the cylindrical part front portion (i.e., a surface of the cylindrical part, which is close to the human body) is preferably 0.2 to 1.0. That is, in this embodiment, the static friction coefficient of a surface of the planar material forming the front portion of the cylindrical part, which is close to the human body, is preferably 0.2 or more and 1.0 or less. Here, the surface of the planar material forming the cylindrical part, which is close to the human body, is a surface of the cylindrical part front portion 15, which may be in contact with the body of the wearer or an underwear worn by the wearer, at the time of wearing the bottom wear shown in Figs. 10 and 11. The means for adjusting the static friction coefficient of the fabric is as described above. Since the static friction coefficient of the surface of the cylindrical part front portion, which is close to the human body, is 0.2 to 1.0, the effect of suppressing a situation in which an underwear worn by a wearer is slid in a wearer's stature direction with respect to a waist portion of the wearer as the wearer moves becomes more remarkable.

The effect of the bottom wear of the second embodiment as well as the bottom wear of the first embodiment in which the static friction coefficient and the stress at elongation are each in a specific range will be described. The static friction coefficient of the inner surface of the cylindrical part rear portion of the waist-encircling section provided in the bottom wear is preferably 0.2 to 1.0, and the stress of the cylindrical part front portion at 10% elongation is preferably 10 N/25 mm or less. Since the bottom wear of the present invention has the above-described characteristics, work for attaching the protection band for lumbago to the cylindrical part is simplified. A supposed mechanism in which the above-described effect is exhibited may be as follows.

That is, when the protection band for lumbago is attached to the cylindrical part, one end of the band portion of the protection band for lumbago is inserted through the slit provided at the center of the cylindrical part front portion shown with symbol 25 in Fig. 1 and symbol 26 in Fig. 8, and at the time of inserting the band to near the center of the waist portion protecting portion (Fig. 24), the other end of the band portion of the protection band for lumbago is similarly inserted into the slit provided at the center of the cylindrical part front portion, so that the protection band for lumbago is attached to the cylindrical part (Fig. 25). Here, the inner surface of the cylindrical part rear portion comes into contact with the surface B of the protection band for lumbago. In this work, the static friction coefficient of the inner surface of the cylindrical part rear portion of the waist-encircling section provided in the bottom wear of the present invention is as small as 0.2 or more and 1.0 or less, and thus the protection band for lumbago smoothly moves without being caught in the cylindrical part, so that attachment work is simplified. In addition, the cylindrical part front portion extends upward appropriately, so that particularly when both ends of the protection band for lumbago are inserted through the same slit, the opening of the slit can be only slightly widened due to the stretchability of the cylindrical part front portion, leading to smoother insertion work.

In the second embodiment as well as the first embodiment, the static friction coefficient of the inner surface of the cylindrical part rear portion provided in the bottom wear of the present invention is preferably 0.2 to 1.0 as described above. Since the static friction coefficient of the inner surface of the cylindrical part rear portion is 1.0 or less, the protection band for lumbago can be caused to smoothly pass without being caught when the band is inserted into the cylindrical part. On the other hand, since the static friction coefficient of the inner surface of the cylindrical part rear portion is 0.2 or more, the following effect is exhibited. That is, it is possible to suppress a situation in which the protection band for lumbago excessively moves in the cylindrical part present in the waist-encircling section of the bottom wear, so that work for inserting the protection band for lumbago into the cylindrical part becomes rather difficult. The static friction coefficient is preferably 0.3 or more, more preferably 0.4 or more because the above-described effect can be further improved. In addition, the static friction coefficient is more preferably 0.8 or less, still more preferably 0.6 or less because the above-described effect can be further improved.

In the second embodiment as well as the first embodiment, the stress of the cylindrical part front portion is preferably 10 N/25 mm or less at 10% elongation in the direction I in the bottom wear of the present invention. The stress of the cylindrical part front portion is 10 N/25 mm or less at 10% elongation in the direction I, and thus when the protection band for lumbago is inserted through the slit present at the substantially central part of the cylindrical part, the opening of the slit is slightly opened due to stretchability of the cylindrical part to facilitate insertion of the protection band for lumbago as described above. The stress of the cylindrical part front portion is more preferably 1 N/25 mm or less at 10% elongation in the direction I because the above-described effect is extremely remarkably exhibited. Here, the direction I of the cylindrical part refers to a direction parallel to the wearer's stature direction. In addition, the lower limit of the stress of the cylindrical part front portion at 10% elongation in the direction I is not particularly limited, but when the cylindrical part front portion is excessively extended, the portion does not completely return to an original length, and strain is apt to remain, so that creases or a feeling of stiffness around the waist may occur. From the viewpoint of the recovery rate from such a condition, the stress of the cylindrical part front portion is preferably 0.2 N/25 mm or more.

In addition, in the second embodiment as well as the first embodiment, the static friction coefficient of a surface of the cylindrical part front portion, which is far from the human body, (i.e., a surface of the cylindrical part front portion on a side opposite to the surface on the wearer side), in the bottom wear of the present invention, is preferably 0.2 to 1.0. That is, when the protection band for lumbago is inserted into the cylindrical part, a surface of the cylindrical part front portion which is far from the human body comes into contact with a surface of the protection band for lumbago on the wearer side (i.e., the surface A of the protection band for lumbago). Thus, for the same reason as described above, since the static friction coefficient of the surface of a part of the cylindrical part front portion, which is far from the human body, is 1.0 or less, the protection band for lumbago can be caused to smoothly pass without being caught when the band is inserted into the cylindrical part. On the other hand, since the static friction coefficient of the inner surface of the cylindrical part rear portion is 0.2 or more, it is possible to suppress a situation in which the protection band for lumbago excessively moves in the cylindrical part present in the waist-encircling section of the bottom wear, so that work for inserting the protection band for lumbago into the cylindrical part becomes rather difficult. The static friction coefficient is preferably 0.3 or more, more preferably 0.4 or more because the above-described effect can be further improved. In addition, the static friction coefficient is more preferably 0.8 or less, still more preferably 0.6 or less because the above-described effect can be further improved.

In addition, in the second embodiment as well as the first embodiment, the static friction coefficient of a surface of the cylindrical part front portion, which is close to the human body, i.e., a surface of the cylindrical part front portion on the wearer side, in the bottom wear of the present invention, is preferably 0.2 to 1.0. Particularly when the bottom wear is worn with the protection band for lumbago attached to the bottom wear of the present invention, the underwear worn by the wearer at this surface comes into close contact with the cylindrical part front portion, but since the static friction coefficient is 1.0 or less, it is possible to suppress a situation in which the bottom wear conforms to the movement of the underwear. On the other hand, since when the positional relationship between the underwear and the bottom wear excessively smoothly moves, the wearer may feel uncomfortable, the static friction coefficient of this surface is preferably 0.2 or more. The static friction coefficient is more preferably 0.3 or more, still more preferably 0.4 or more because the above-mentioned effect can be further improved. In addition, the static friction coefficient of this surface is more preferably 0.8 or less, still more preferably 0.6 or less because the above-described effect can be further improved.

In the bottom wear of the first embodiment, the cloth of bottom wear is a cylindrical part rear portion. Preferably, the planar material of the front portion forming the cylindrical part is continuously or intermittently connected to the cloth of bottom wear at the upper part. This connection is established preferably at the upper end in the transverse direction of the cylindrical part or in the vicinity thereof in the substantially vertical direction. The upper part of the planar material means a portion including the upper end of the planar material and a portion situated below the upper end of the planar material. It is desirable that the planar material of the cylindrical part front portion be continuously or intermittently connected to the cloth of bottom wear at the lower part. This connection is established preferably at the lower end in the transverse direction of the cylindrical part, or in the vicinity thereof. The lower part of the planar material means a portion including the lower end of the planar material and a portion situated above the lower end of the planar material. When the cloth of bottom wear is used for about a half of the cylindrical part, it is possible to form a compact cylindrical part as compared to the bottom wear of the second embodiment.

On the other hand, in the bottom wear of the second embodiment, a planar material other than the cloth of bottom wear is used. Preferably, the planar material is continuously or intermittently connected to the cloth of bottom wear at the upper part of the cylindrical part. This connection is established preferably at the upper end in the transverse direction of the cylindrical part or in the vicinity thereof in the substantially vertical direction. The upper part of the planar material means a portion including the upper end of the planar material and a portion situated below the upper end of the planar material. Further, it is desirable that the planar material be continuously or intermittently connected to the back portion of the waist-encircling section in the waist-encircling direction at the lower part of the planar material. This connection is established preferably at the lower end in the transverse direction of the cylindrical part, or in the vicinity thereof. The lower part of the planar material means a portion including the lower end of the planar material and a portion situated above the lower end of the planar material. In this way, the cylindrical part is suspended at the upper part of the cylindrical part, and the lower part of the cylindrical part is partially connected, so that at the time when the protection band for lumbago is inserted into the bottom wear, the silhouette of the appearance from behind is clearly seen.

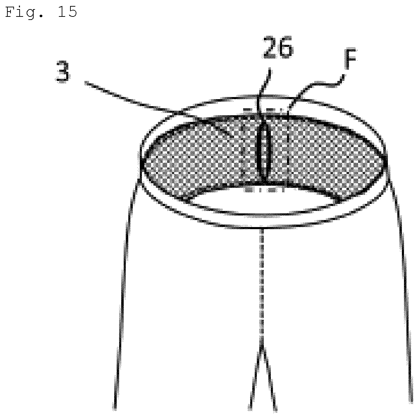

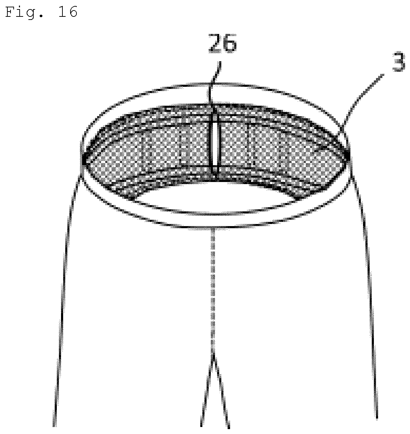

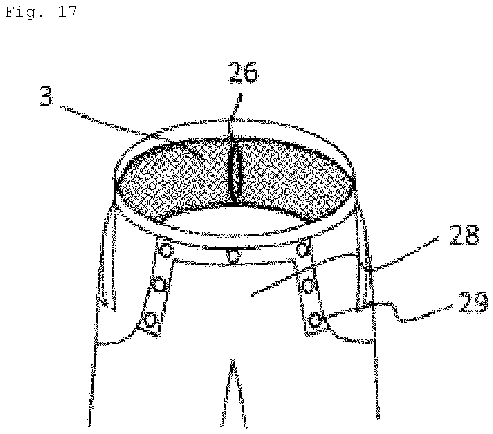

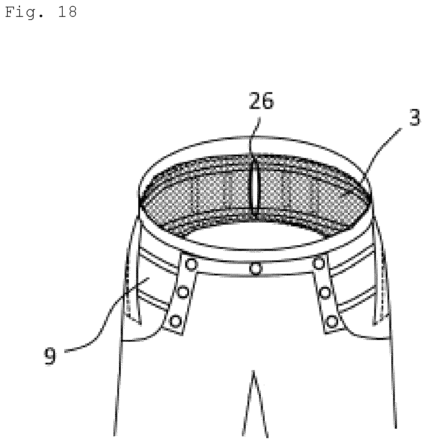

In addition, when the bottom wear of the present invention is worn, the protection band for lumbago is inserted into the cylindrical part, and the right end is locked with the left end of the protection band for lumbago, whereby adjustment can be made so that the wearer feels a moderate abdominal pressure at the waist portion (pelvic position). Here, the appearance of the bottom wear according to the present invention is not particularly limited, and the locking portion between the right end and the left end of the protection band for lumbago may be visible or hidden from outside the wearer. Examples of the appearance of the bottom wear having a structure in which the locking portion between the right end and the left end of the protection band for lumbago is visible include those shown in Figs. 6 and 10. On the other hand, examples of the appearance of the bottom wear having a structure in which the locking portion between the right end and the left end of the protection band for lumbago is hidden include that of the bottom wear shown in Fig. 15, and Fig. 16 shows an appearance where the protection band for lumbago is attached to the bottom wear. Thus, the bottom wear may have a structure in which the protection band for lumbago is completely locked inside the bottom wear, and completely stored inside the bottom wear. Examples of the bottom wear having a structure in which the protection band for lumbago is partially hidden include the bottom wear shown in Fig. 17, and Fig. 18 shows an appearance where the protection band for lumbago is attached to the bottom wear. Thus, a cover cloth 28 provided on the front surface of the bottom wear is locked with the front surface of the bottom wear by a button shown with symbol 29 in Fig. 17, etc., whereby the protection band for lumbago can be partially hidden. Further, the bottom wear shown in Fig. 17 has a structure in which as shown in Fig. 19, the bottom wear can be worn in a state in which the locking portion between the right end and the left end of the protection band for lumbago is not hidden but exposed to the front surface, and partially hiding the protection band for lumbago or exposing the protection band for lumbago to the front surface without being hidden can be selected depending on a usage situation or a wearer's intention. Thus, since the bottom wear has a structure in which the appearance of the bottom wear at the time of attaching the protection band for lumbago can be selected depending on a usage situation or a wearer's intention, it is possible to meet various needs of the wearer with one pattern.

### EXAMPLES

Hereinafter, the present invention will be described in further detail by way of examples.

### (Measurement methods)

### (Static friction coefficient)

The static friction coefficient of a fabric sample was measured in accordance with Inclination Method in JIS P 8147: 1994 3.2. Three test pieces each having a width of 25 mm and a length of 130 mm were prepared. Next, one of the three test pieces was attached to a weight of 872 g in a slip inclination angle measuring apparatus. On the other hand, a friction target having a width of 120 mm and a length of 200 mm was attached to the slip inclination angle measuring apparatus, and the weight with the test piece was placed in such a manner that the measurement surface of the test piece was in contact with the friction target, and the lengthwise direction of the test piece was coincide with the sliding direction of the slip inclination angle measuring apparatus. The degree of inclination was increased under the condition of an inclination angle of 3°/second, the inclination angle at the time when the weight fell down was read, and the tangent (tan θ) of the inclination angle was defined as a static friction coefficient. For the other two test pieces, the static friction coefficient was measured in the same manner as described above, and an average of the measured values of the static friction coefficients of the three test pieces was defined as a static friction coefficient of the fabric sample. Here, the test piece is sampled in such a manner that the lengthwise direction of the test piece is identical to the direction I of the cylindrical part front portion. In addition, as the friction target, standard cotton (No. 3-1: manufactured by Japanese Standards Association) is used, and the direction of the fabric of this standard cotton may be a warp direction or weft direction.

### (Stress at 10% elongation)

The stress at 10% elongation of the fabric sample was measured using Autograph AG-50kNG manufactured by Shimadzu Corporation. Specifically, five test pieces each having a width of 25 mm and a length of 220 mm were prepared. Next, one of the five test pieces was attached to the above-described measuring equipment at a chuck distance of 100 mm, and pulled at a tensile speed of 300 mm/min until the test piece was broken. The other four test pieces were subjected to the same treatment as described above, and the stress of each of the five test pieces at 10% elongation was measured. The "stress at 10% elongation" mentioned here is an average of the elongation stresses of the five fabrics at an elongation of 10% as measured by the above-described method. Here, the test piece is sampled in such a manner that the lengthwise direction of the test piece is identical to the direction I of the cylindrical part front portion.

### (Evaluation Method)

### (Amount of time required for work for attaching protection band for lumbago)

The amount of time (seconds) required for 20 test subjects to attach the protection band for lumbago to the bottom wear of each of examples and comparative examples was measured using a stopwatch. The 20 test subjects each performed work for attaching a protection band for lumbago three times, an average of the amounts of time (seconds) for each test subject was calculated, and an average for the 20 test subjects was defined as the amount of time required for work for attaching the protection band for lumbago.

### (Examples)

### (Example 1)

Polyethylene terephthalate yarns of 167 dtex and spun yarns with 65% of polyester and 35% (mass ratio) of cotton were used as warp threads, and polyethylene terephthalate yarns of 167 dtex were used as weft threads to weave a warp double twill fabric at a warp density of 154 threads/2.54 cm and a weft density of 64 threads/2.54 cm, thereby obtaining a cloth A for cloth of bottom wear with 90% of polyester and 10% of cotton.

Subsequently, nylon yarns of 78 dtex and polyurethane elastic fiber of 310 dtex were used to knit a fabric, thereby obtaining a power net cloth with 82% of nylon and 18% of polyurethane (hereinafter, the power net cloth is referred to as a "cloth D").

The cloth A for cloth of bottom wear bottom was used as a cloth of bottom wear to prepare a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth D. In addition, only one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.88. In addition, the slit is such that the edge of the opening of the slit is sandwiched between cloths to surround the opening, followed by stitching. A protection band for lumbago with a configuration as shown in Figs. 4 and 5 was prepared, Mold Magic (registered trademark) (manufactured by Kuraray Fastening Co., Ltd.) of extrusion-molded type was used as a hook-and-loop male surface for the locking portion, and Ring Fastener (manufacture by SEIHOU Co., Ltd.) was used as a female surface. The protection band for lumbago was defined as a protection band for lumbago 1. The protection band for lumbago 1 was attached to the cylindrical part of the bottom wear as shown in Fig. 6, and the amount of time required for this work was measured. Table 1 shows the physical properties of the fabric forming the cylindrical part and the evaluation results of the bottom wear.

### (Example 2)

A cloth of bottom wear was prepared using the same cloth A as used in Example 1, and further, the same cloth D as used in Example 1 was used to prepare a cylindrical part, thereby preparing a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth D. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.94. The protection band for lumbago 1 having a configuration as shown in Figs. 4 and 5 was attached to the cylindrical part of the bottom wear as in Fig. 6, and the amount of time required for this work was measured. Table 1 shows the physical properties of the fabric forming the cylindrical part and the evaluation results of the bottom wear.

### (Example 3)

A cloth of bottom wear was prepared using the same cloth A as used in Example 1, and further, the same cloth D as used in Example 1 was used to prepare a cylindrical part, thereby preparing a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth D. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.75. The protection band for lumbago 1 having a configuration as shown in Figs. 4 and 5 was attached to the cylindrical part of the bottom wear as in Fig. 6, and the amount of time required for this work was measured. Table 1 shows the physical properties of the fabric forming the cylindrical part and the evaluation results of the bottom wear.

### (Example 4)

A cloth of bottom wear was prepared using the same cloth A as used in Example 1, and further, the same cloth D as used in Example 1 was used to prepare a cylindrical part, thereby preparing a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth D. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.96. The protection band for lumbago 1 having a configuration as shown in Figs. 4 and 5 was attached to the cylindrical part of the bottom wear as in Fig. 6, and the amount of time required for this work was measured. Table 1 shows the physical properties of the fabric forming the cylindrical part and the evaluation results of the bottom wear.

### (Example 5)

A cloth of bottom wear was prepared using the same cloth A as used in Example 1, and further, the same cloth D as used in Example 1 was used to prepare a cylindrical part, thereby preparing a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth D. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 1.00. The protection band for lumbago 1 having a configuration as shown in Figs. 4 and 5 was attached to the cylindrical part of the bottom wear as in Fig. 6, and the amount of time required for this work was measured. Table 1 shows the physical properties of the fabric forming the cylindrical part and the evaluation results of the bottom wear.

### (Example 6)

Nylon yarns of 44 dtex and polyurethane elastic fiber of 44 dtex were used to knit a tricot, thereby obtaining a tricot cloth with 70% of nylon and 30% (mass ratio) of polyurethane (hereinafter, the tricot cloth is referred to as a "cloth E"). The same cloth A as used for the bottom wear in Example 1, and the cloth E were used to prepare a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth E. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 6, and the amount of time required for this work was measured. Table 2 shows the physical properties of the fabric forming the cylindrical part and the evaluation results of the bottom wear.

### (Example 7)

PTFE fiber of 3.3 dtex was used as warp threads to prepare a twill fabric with 100% of PTFE (areal weight: 522 g/m²), and the fabric was defined as a cloth B for cloth of bottom wear. The cloth B was used as the bottom wear cloth on the back portion in the waist-encircling section of the cloth of bottom wear, and the same cloth A as used in Example 1 was used for other portions of the cloth of the bottom wear to prepare a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth B, and the fabric of the cylindrical part front portion (planar material) is the cloth D which is identical to that used in Example 1. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 6, and the amount of time required for this work was measured. Table 2 shows the physical properties of the fabric forming the cylindrical part and the evaluation results of the bottom wear.

### (Example 8)

PPT composite fiber of 84 dtex and spun yarns with 100% of cotton were used to knit a tricot at 28 gauge, thereby obtaining a tricot cloth (hereinafter, the tricot cloth is referred to as a "cloth F"). The same cloth A as used for the bottom wear in Example 1, and the cloth F were used to prepare a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth F. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 6, and the amount of time required for this work was measured. Table 2 shows the physical properties of the fabric forming the cylindrical part and the evaluation results of the bottom wear.

### (Example 9)

A knit cloth with 100% of cotton, which was subjected to silicon dot processing with a diameter of 1 mm at intervals of 1 mm (the static friction coefficient of a surface subjected to silicon dot processing is 0.91) was defined as a cloth C. The cloth C was used as the bottom wear cloth on the back portion in the waist-encircling section of the cloth of bottom wear, and the same cloth A as used in Example 1 was used for other portions of the cloth of the bottom wear to prepare a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth C, and the fabric of the cylindrical part front portion (planar material) is the cloth D which is identical to that used in Example 1. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 6, and the amount of time required for this work was measured. Table 2 shows the physical properties of the fabric forming the cylindrical part and the evaluation results of the bottom wear.

### (Example 10)

A cloth of bottom wear was prepared using the same cloth A as used in Example 1, and further, the same cloth D as used in Example 1 was used to prepare a cylindrical part, thereby preparing a bottom wear having an appearance as shown in Figs. 8 and 2 and an inner part as shown in Fig. 9. The cylindrical part was formed in the following manner: the same cloth D as in Example 1 was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the back portion of the waist-encircling section. The fabric of the cylindrical part rear portion in Fig. 9 is the cloth D, and the fabric of the cylindrical part front portion (planar material) is also the cloth D. In addition, one slit as shown in Fig. 8 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 10, and the amount of time required for this work was measured. Table 2 shows the physical properties of the fabric forming the cylindrical part and the evaluation results of the bottom wear.

### (Example 11)

A cloth of bottom wear was prepared using the same cloth A as used in Example 1, and further, the same cloth E as used in Example 6 was used to prepare a cylindrical part, thereby preparing a bottom wear having an appearance as shown in Figs. 8 and 2 and an inner part as shown in Fig. 9. The cylindrical part was formed in the following manner: the same cloth E as in Example 1 was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the back portion of the waist-encircling section. The fabric of the cylindrical part rear portion in Fig. 9 is the cloth E, and the fabric of the cylindrical part front portion (planar material) is also the cloth E. In addition, a slit as shown in Fig. 8 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 10, and the amount of time required for this work was measured. Table 2 shows the physical properties of the fabric forming the cylindrical part and the evaluation results of the bottom wear.

### (Example 12)

A cloth of bottom wear was prepared using the same cloth A as used in Example 1, and further, the same cloth F as used in Example 8 was used to prepare a cylindrical part, thereby preparing a bottom wear having an appearance as shown in Figs. 8 and 2 and an inner part as shown in Fig. 9. The cylindrical part was formed in the following manner: the same cloth F as in Example 1 was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the back portion of the waist-encircling section. The fabric of the cylindrical part rear portion in Fig. 9 is the cloth F, and the fabric of the cylindrical part front portion (planar material) is also the cloth F. In addition, one slit as shown in Fig. 8 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 10, and the amount of time required for this work was measured. Table 3 shows the physical properties of the fabric forming the cylindrical part and the evaluation results of the bottom wear.

### (Example 13)

In a 30 gauge double tricot knitting machine, polyurethane elastic yarns of 33 dtex were prepared for a front reed, a nylon original yarns of 22 dtex/7 filaments were prepared for a back reed, and a warp knitted fabric having knitted structures at both sides and a polyurethane elastic yarn content of 53% was knitted in the following structure.
Front reed: 1-0/1-2/2-3/2-1//
Back reed: 2-3/1-1/1-0/2-2//

In this way, a tricot cloth having different static friction coefficients on the front and the back (hereinafter, this tricot cloth is referred to as a cloth G) is obtained. In the tricot cloth, a knit loop was formed with only an elastic yarn to form a warp knitted fabric on one surface of the double knitted structure, a knit loop was formed by use of an elastic yarn and an inelastic yarn in combination to form a warp knitted fabric on the other surface, and the surface and the back surface had static friction coefficients of 0.60 and 0.39, respectively.

A cloth of bottom wear was prepared using the same cloth A as used in Example 1, and further, the cloth G was used to prepare a cylindrical part and prepare a bottom wear having an appearance as shown in Figs. 8 and 2 and an inner part as shown in Fig. 9. The cylindrical part was formed in the following manner: the cloth G was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the back portion of the waist-encircling section. In accordance with the characteristics of the cloth, a surface of the cloth G, which has a static friction coefficient of 0.39, is disposed on the inner surface of the cylindrical part rear portion, and a surface of the cloth G, which has a static friction coefficient of 0.60, is disposed on the outer surface (a surface of a part close to the human body) of the cylindrical part front portion. In addition, one slit as shown in Fig. 8 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 10, and the amount of time required for this work was measured. Table 3 shows the physical properties of the fabric forming the cylindrical part and the evaluation results of the bottom wear.

### (Example 14)

A cloth of bottom wear was prepared using the same cloth A as used in Example 1, and further, the same cloth G as used in Example 13 was used to prepare a cylindrical part, thereby preparing a bottom wear having an appearance as shown in Figs. 8 and 2 and an inner part as shown in Fig. 9. However, the cloth G is reversed in the front and the back, and in accordance with the characteristics of the cloth, a surface of the cloth G, which has a static friction coefficient of 0.60, is disposed on the inner surface of the cylindrical part rear portion, and a surface of the cloth G, which has a static friction coefficient of 0.39, is disposed on the outer surface (a surface of a part close to the human body) of the cylindrical part front portion. In addition, one slit as shown in Fig. 8 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 10, and the amount of time required for this work was measured. Table 3 shows the physical properties of the fabric forming the cylindrical part and the evaluation results of the bottom wear.

### (Example 15)

A cloth of bottom wear was prepared using the same cloth A as used in Example 1, and further, the same cloth D as used in Example 1 was used to prepare a cylindrical part, thereby preparing a bottom wear having an appearance as shown in Figs. 20 and 2 and an inner part as shown in Fig. 9. The cylindrical part was formed in the following manner: the same cloth D as in Example 1 was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the back portion of the waist-encircling section. The fabric of the cylindrical part rear portion in Fig. 9 is the cloth D, and the fabric of the cylindrical part front portion (planar material) is also the cloth D. In addition, as the cylindrical part, a cylindrical part was divided at the center into two equal parts with a spacing of 1 cm provided therebetween as shown in Fig. 20. In the cylindrical part, the width of the cylindrical part (W1) is equal to the width of a cylindrical part dividing portion 30 in the longitudinal direction (W2), i.e., the ratio (W2/W1) is 1.00. The protection band for lumbago 1 having a configuration as shown in Figs. 4 and 5 was attached to the cylindrical part of the bottom wear, and the amount of time required for this work was measured. Table 3 shows the physical properties of the fabric forming the cylindrical part and the evaluation results of the bottom wear.

### (Example 16)

A cloth of bottom wear was prepared using the same cloth A as used in Example 1, and further, the same cloth A as used in Example 1 was used to prepare a cylindrical part, thereby preparing a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth A. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 6, and the amount of time required for this work was measured. Table 3 shows the physical properties of the fabric forming the cylindrical part and the evaluation results of the bottom wear.

### (Example 17)

Three anti-slipping tapes having a length of 14 cm and a width of 25 mm and having two high friction bodies continuously disposed from one end portion to the other end portion in the longitudinal direction (800 A manufactured by Ando Tape Co., Ltd.; surface to which a large number of elastic yarns are exposed, has a static friction coefficient of 1.07) were prepared. To the back portion of the waist-encircling section of the cloth of bottom wear with the cloth A used in the same manner as in Example 1, these anti-slipping tapes were stitched and bonded at total three positions: the central part of the back portion of the waist-encircling section and portions at a distance of 8 cm on the left and the right from the central part. The longitudinal direction of the anti-slipping tape was made substantially parallel to the direction I. This cloth of bottom wear and the same cloth D as used in Example 1 were used to prepare a bottom wear having an appearance as shown in Figs. 1 and 2 and an inner part as shown in Fig. 3. The fabric of the cylindrical part rear portion in Fig. 3 is the cloth A to which three anti-slipping tapes are stitched and bonded, and the fabric of the cylindrical part front portion (planar material) is the same cloth D as in Example 1. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.88. The protection band for lumbago 1 having a configuration as shown in Figs. 4 and 5 was attached to the cylindrical part of the bottom wear as in Fig. 6, and the amount of time required for this work was measured. Table 3 shows the physical properties of the fabric forming the cylindrical part and the evaluation results of the bottom wear.

### (Comparative Example 1)

A cloth of bottom wear was prepared using the same cloth A as used in Example 1, and further, the same cloth D as used in Example 1 was used to prepare a cylindrical part, thereby preparing a bottom wear having an appearance as shown in Figs. 22 and 2 and an inner part as shown in Fig. 9. The cylindrical part was formed in the following manner: the same cloth D as in Example 1 was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the back portion of the waist-encircling section. The fabric of the cylindrical part rear portion in Fig. 9 is the cloth D, and the fabric of the cylindrical part front portion (planar material) is also the cloth D. In addition, a slit was not provided in cylindrical part front portion. The protection band for lumbago 1 having a configuration as shown in Figs. 4 and 5 was attached to the cylindrical part of the bottom wear, and the amount of time required for this work was measured. Table 4 shows the physical properties of the fabric forming the cylindrical part and the evaluation results of the bottom wear.

The time required to insert the protection band for lumbago was 25 seconds, so that much time is required for attaching the protection band for lumbago to the bottom wear. This may be because as described above, the bottom wear having a structure as shown in Fig. 22 requires passage of the protection band for lumbago from one opening to the other opening of the cylindrical part, and much time was consumed because of the long band passage distance.

### (Comparative Example 2)

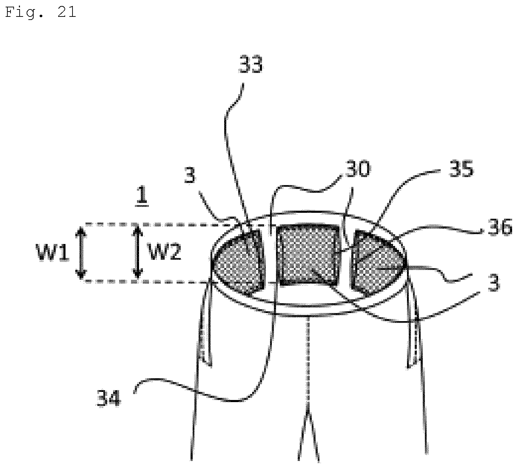

A cloth of bottom wear was prepared using the same cloth A as used in Example 1, and further, the same cloth D as used in Example 1 was used to prepare a cylindrical part, thereby preparing a bottom wear having an appearance as shown in Figs. 21 and 2 and an inner part as shown in Fig. 9. The cylindrical part was formed in the following manner: the same cloth D as in Example 1 was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the back portion of the waist-encircling section. The fabric of the cylindrical part rear portion in Fig. 9 is the cloth D, and the fabric of the cylindrical part front portion (planar material) is also the cloth D. Further, the cylindrical part is provided with two slits as shown in Fig. 21. That is, the slit of the cylindrical part extends to the rear portion of the cylindrical part, and has two cylindrical portions 30 which provides a form of the slit. A cylindrical part was prepared in which the width of the cylindrical part (W1) is equal to the width of a cylindrical part dividing portion 30 in the longitudinal direction (W2), i.e., the ratio (W2/W1) is 1.00. The protection band for lumbago 1 having a configuration as shown in Figs. 4 and 5 was attached to the cylindrical part of the bottom wear, and the amount of time required for this work was measured. Table 4 shows the physical properties of the fabric forming the cylindrical part and the evaluation results of the bottom wear.

The time required to insert the protection band for lumbago was 24 seconds, so that much time is required for attaching the protection band for lumbago to the bottom wear. In the bottom wear having a structure as in Fig. 21, the slit of the cylindrical part extends to the rear portion of the cylindrical part, and two cylindrical part dividing portions 30 are provided as a form of the slit, so that it is necessary for the band to pass through four openings: a cylindrical part opening C, a cylindrical part opening D, a cylindrical part opening E and a cylindrical part opening F, resulting in expansion of time-consuming work. Further, since each cylindrical part opening moves freely, time-consuming work occurred such that after the support band for lumbago passed through one cylindrical part opening, the opening was widened by the hand at the time of inserting the band through the other cylindrical part openings.

### (Comparative Example 3)

A cloth of bottom wear was prepared using the same cloth A as used in Example 1, and further, the same cloth D as used in Example 1 was used to prepare a cylindrical part, thereby preparing a bottom wear having an appearance as shown in Figs. 8 and 2 and an inner part as shown in Fig. 9. The cylindrical part was formed in the following manner: the same cloth D as in Example 1 was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the back portion of the waist-encircling section. The fabric of the cylindrical part rear portion in Fig. 9 is the cloth D, and the fabric of the cylindrical part front portion (planar material) is also the cloth D. In addition, one slit as shown in Fig. 8 was provided at the central part of the cylindrical part front portion, a cylindrical part was prepared in such a manner that the ratio (W2/W1) of the width of the slit in the longitudinal direction (W2) to the width of the cylindrical part (W1) was 0.68. The protection band for lumbago 1 having a configuration as shown in Figs. 4 and 5 was attached to the cylindrical part of the bottom wear as in Fig. 10, and the amount of time required for this work was measured. Table 4 shows the physical properties of the fabric forming the cylindrical part and the evaluation results of the bottom wear.

The time required to insert the protection band for lumbago was 21 seconds, so that much time is required for attaching the protection band for lumbago to the bottom wear. This may be because in a bottom wear having a structure in which the ratio (W2/W1) of the width of the slit in the longitudinal direction (W2) to the width of the cylindrical part (W1) is 0.68, the width of the cylindrical part (W1) is not sufficient with respect to the back width of the protection band for lumbago, so that time-consuming work occurred such that at the time of inserting the band, the opening was widened by the hand to give support.

### (Comparative Example 4)

A cloth of bottom wear was prepared using the same cloth A as used in Example 1, and further, the same cloth D as used in Example 1 was used to prepare a cylindrical part, thereby preparing a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth D. In addition, one slit as shown in Fig. 1 was provided at the central part of the cylindrical part front portion, a cylindrical part was prepared in such a manner that the ratio (W2/W1) of the width of the slit in the longitudinal direction (W2) to the width of the cylindrical part (W1) was 0.68. The protection band for lumbago 1 having a configuration as shown in Figs. 4 and 5 was attached to the cylindrical part of the bottom wear as in Fig. 6, and the amount of time required for this work was measured. Table 4 shows the physical properties of the fabric forming the cylindrical part and the evaluation results of the bottom wear.

The time required to insert the protection band for lumbago was 21 seconds, so that much time is required for attaching the protection band for lumbago to the bottom wear. This may be because in a bottom wear having a structure in which the ratio (W2/W1) of the width of the slit in the longitudinal direction (W2) to the width of the cylindrical part (W1) is 0.68, the width of the cylindrical part (W1) is not sufficient with respect to the back width of the protection band for lumbago, so that time-consuming work occurred such that at the time of inserting the band, the opening was widened by the hand to give support.

**[Table 1]**

| | | | | Example 1 | Example 2 | Example 3 | Example 4 | Example 5 |
|---|---|---|---|---|---|---|---|---|
| Bottom wear | Configuration | Cloth of bottom wear | | Cloth A | Cloth A | Cloth A | Cloth A | Cloth A |
| | | Configuration of cylindrical part: | | Cylindrical part according to first embodiment of invention | Cylindrical part according to first embodiment of invention | Cylindrical part according to first embodiment of invention | Cylindrical part according to first embodiment of invention | Cylindrical part according to first embodiment of invention |
| | | Cylindrical part rear portion | Cloth | Cloth A | Cloth A | Cloth A | Cloth A | Cloth A |
| | | | Static friction coefficient of inner surface | 0.46 | 0.46 | 0.46 | 0.46 | 0.46 |
| | | Cylindrical part front portion | Cloth | Cloth D | Cloth D | Cloth D | Cloth D | Cloth D |
| | | | Stress at 10% elongation in direction I (N/25 mm) | 0.50 | 0.50 | 0.50 | 0.50 | 0.50 |
| | | | Static friction coefficient of surface of part far from human body | 0.53 | 0.53 | 0.53 | 0.53 | 0.53 |
| | | Slit | Number of slits | 1 | 1 | 1 | 1 | 1 |
| | | | Ratio of W2 to W1 (W2/W1) | 0.88 | 0.94 | 0.75 | 0.96 | 1.00 |
| Amount of time (seconds) required for inserting protection band for lumbago | | | | 10 | 9 | 13 | 9 | 12 |

**[Table 2]**

| | | | | Example 6 | Example 7 | Example 8 | Example 9 | Example 10 | Example 11 |
|---|---|---|---|---|---|---|---|---|---|
| Bottom wear | Configuration | Cloth of bottom wear | | Cloth A | Cloth B | Cloth A | Cloth C | Cloth A | Cloth A |
| | | Configuration of cylindrical part: | | Cylindrical part according to first embodiment of invention | Cylindrical part according to first embodiment of invention | Cylindrical part according to first embodiment of invention | Cylindrical part according to first embodiment of invention | Cylindrical part according to second embodiment of invention | Cylindrical part according to second embodiment of invention |
| | | Cylindrical part rear portion | Cloth | Cloth A | Cloth B | Cloth A | Cloth C | Cloth D | Cloth E |
| | | | Static friction coefficient of inner surface | 0.46 | 0.27 | 0.46 | 0.91 | 0.53 | 0.49 |
| | | Cylindrical part front portion | Cloth | Cloth E | Cloth D | Cloth F | Cloth D | Cloth D | Cloth E |
| | | | Stress at 10% elongation in direction I (N/25 mm) | 0.65 | 0.50 | 10.00 | 0.50 | 0.50 | 0.65 |
| | | | Static friction coefficient of surface of part far from human body | 0.49 | 0.53 | 0.50 | 0.53 | 0.53 | 0.49 |
| | | Slit | Number of slits | 1 | 1 | 1 | 1 | 1 | 1 |
| | | | Ratio of W2 to W1 (W2/W1) | 0.88 | 0.88 | 0.88 | 0.88 | 0.88 | 0.88 |
| Amount of time (seconds) required for inserting protection band for lumbago | | | | 11 | 10 | 14 | 14 | 9 | 11 |

**[Table 3]**

| | | | | Example 12 | Example 13 | Example 14 | Example 15 | Example 16 | Example 17 |
|---|---|---|---|---|---|---|---|---|---|
| Bottom wear | Configuration | Cloth of bottom wear | | Cloth A | Cloth A | Cloth A | Cloth A | Cloth A | Cloth A |
| | | Configuration of cylindrical part: | | Cylindrical part according to second embodiment of invention | Cylindrical part according to second embodiment of invention | Cylindrical part according to second embodiment of invention | Cylindrical part similar to cylindrical part in bottom wear shown in Fig. 20 | Cylindrical part similar to cylindrical part according to first embodiment of invention | Cylindrical part similar to cylindrical part according to first embodiment of invention |
| | | Cylindrical part rear portion | Cloth | Cloth F | Cloth G | Cloth G | Cloth D | Cloth A | Cloth A to which three anti-slipping tapes are stitched and bonded |
| | | | Static friction coefficient of inner surface | 0.50 | 0.39 | 0.60 | 0.53 | 0.46 | 1.07 |
| | | Cylindrical part front portion | Cloth | Cloth F | Cloth G | Cloth G | Cloth D | Cloth A | Cloth D |
| | | | Stress at 10% elongation in direction I (N/25 mm) | 10.00 | 0.65 | 0. 65 | 0.50 | 12.00 | 0.50 |
| | | | Static friction coefficient of surface of part far from human body | 0.50 | 0.39 | 0.60 | 0.53 | 0.46 | 0.53 |
| | | Slit | Number of slits | 1 | 1 | 1 | 1 | 1 | 1 |
| | | | Ratio of W2 to W1 (W2/W1) | 0.88 | 0.88 | 0.88 | 1.00 | 0.88 | 0.88 |
| Amount of time (seconds) required for inserting protection band for lumbago | | | | 13 | 12 | 13 | 8 | 16 | 19 |

**[Table 4]**

| | | | | Comparative Example 1 | Comparative Example 2 | Comparative Example 3 | Comparative Example 4 |
|---|---|---|---|---|---|---|---|
| Bottom | Configuration | Cloth of bottom wear | | Cloth A | Cloth A | Cloth A | Cloth A |
| | | Configuration of cylindrical part: | | Cylindrical part similar to cylindrical part according to second embodiment of invention | Cylindrical part similar to cylindrical part in bottom wear shown in Fig. 21 | Cylindrical part similar to cylindrical part according to second embodiment of invention | Cylindrical part similar to cylindrical part according to first embodiment of invention |
| | | Cylindrical part rear portion | Cloth | Cloth D | Cloth D | Cloth D | Cloth A |
| | | | Static friction coefficient of inner surface | 0.53 | 0.53 | 0.53 | 0.46 |
| | | Cylindrical part front portion | Cloth | Cloth D | Cloth D | Cloth D | Cloth D |
| | | | Stress at 10% elongation in direction I (N/25 mm) | 0.50 | 0.50 | 0.50 | 0.50 |
| | | | Static friction coefficient of surface of part far from human body | 0.53 | 0.53 | 0.53 | 0.53 |
| | | Slit | Number of slits | 0 | 2 | 1 | 1 |
| | | | Ratio of W2 to W1 (W2/W1) | - | 1.00 | 0.68 | 0.68 |
| Amount of time (seconds) required for inserting protection band for lumbago | | | | 25 | 24 | 21 | 21 |

### DESCRIPTION OF REFERENCE SIGNS

1: Bottom wear
2: Waist-encircling section
3: Cylindrical part
4: Waist-encircling direction
5: Bottom wear cloth on back portion in waist-encircling section
6: Cylindrical part front portion
7: Locking portion
8: Locking portion
9: Protection band for lumbago
10: Waist protecting portion
11: Belt portion
12: First fixing portion
13: Second fixing portion
14: Bone
15: Cylindrical part front portion
15': Cylindrical part rear portion
16: Locking portion
17: Locking portion
18: Back portion
19: Front
20: Rear
21: Space for placement of protection band for lumbago
22: Inner surface of cylindrical part rear portion
23: Outer surface of cylindrical part front portion
24: Cloth of bottom wear
25: Slit
26: Slit
27: Surface of part of cylindrical part front portion which is far from the human body
28: Cover cloth on front surface of bottom wear
29: Button
30: Cylindrical part dividing portion
31: Cylindrical part opening A
32: Cylindrical part opening B
33: Cylindrical part opening C
34: Cylindrical part opening D
35: Cylindrical part opening E
36: Cylindrical part opening F
I: Transverse direction of cylindrical part

## Claims

1. A bottom wear comprising a waist-encircling section, wherein
a cylindrical part including a cloth of bottom wear and a planar material different from the cloth, or
a cylindrical part including a planar material different from the cloth of bottom wear is present at a back portion of the waist-encircling section and inside the bottom wear,
the cylindrical part is disposed in such a manner that the axis direction of the cylindrical part is substantially parallel to the waist-encircling direction of the waist-encircling section,
the cylindrical part includes only one slit satisfying the following conditions (1) and (2), and
the slit is provided at the front portion of the cylindrical part and the substantially central part of the cylindrical part in a longitudinal direction:
(1) the slit is provided in such a manner that the longitudinal direction of the slit is substantially orthogonal to the waist-encircling direction; and
(2) the ratio (W2/W1) of the width (W2) of the slit in the longitudinal direction to the width (W1) of the cylindrical part in a direction orthogonal to the waist-encircling direction is 0.70 to 1.00.

2. The bottom wear according to claim 1, wherein the static friction coefficient of the inner surface of the rear portion of the cylindrical part of the bottom wear is 0.2 to 1.0, and the stress at the front portion of the cylindrical part is 10 N/25 mm or less at 10% elongation of the cylindrical part in the width direction.

3. The bottom wear according to claim 1 or 2, wherein the static friction coefficient of the surface of a part of the front portion of the cylindrical part, which is far from a human body, is 0.2 to 1.0.

4. The bottom wear according to any one of claims 1 to 3, wherein the static friction coefficient of the surface of a part of the front portion of the cylindrical part, which is close to a human body, is 0.2 to 1.0.

5. The bottom wear according to any one of claims 1 to 4, wherein the cylindrical part includes a cloth of bottom wear and a planar material different from the cloth, and
the upper part and the lower part of the planar material are each continuously or intermittently connected to the cloth of bottom wear.

6. The bottom wear according to any one of claims 1 to 4, wherein the cylindrical part includes a planar material different from the cloth of bottom wear, and
the upper part and the lower part of the cylindrical part are each continuously or intermittently connected to the cloth of bottom wear.

7. The bottom wear according to any one of claims 1 to 6, wherein the planar material is a cloth.

8. A bottom wear with a band which includes the bottom wear set forth in any one of claims 1 to 7, and a protection band for lumbago.
